# EUROPEAN PATENT SPECIFICATION

(11) **EP 2 294 012 B1**
(45) Date of publication and mention of the grant of the patent: **09.07.2014**
(21) Application number: 09743658.8
(22) Date of filing: 07.05.2009
(51) Int. Cl.: C01G 3/00, A61K 31/375, A61K 31/395, A61K 31/437, A61K 31/496, A61K 31/765, A61K 33/04, A61K 33/14, A61K 33/42, A61K 45/06, A61P 1/10, A61K 31/00, A61P 1/00

(54) **Administration of a bowel cleanser and an antibiotic for the treatment of bowel disease**
Verabreichung eines Darmreinigers und eines Antibiotikums zur Behandlung von Darmerkrankung
Administration d'un nettoyant d'intestin et d'un antibiotique pour le traitement de maladie intestinale

(30) Priority: 07.05.2008 US 51341 P
(43) Date of publication of application: 16.03.2011
(73) Proprietor: Salix Pharmaceuticals, Ltd., Morrisville, NC 27560 (US)
(72) Inventor: JOHNSON, Lorin, Palo Alto, CA 94301 (US); FORBES, William, Raleigh, NC 27614 (US); PATTON, Stephana, San Francisco, CA 94133 (US)
(74) Representative: Adam, Holger
(86) International application number: PCT/US2009/043138
(87) International publication number: WO 2009/137672

(56) References cited:
- WO-A1-02/07741
- WO-A1-99/16454
- WO-A1-2004/037292
- WO-A1-2005/051361
- WO-A1-2006/122104
- WO-A2-01/11077
- WO-A2-03/075852
- WO-A2-2004/017962
- WO-A2-2006/102536
- WO-A2-2007/103448
- WO-A2-2009/052255
- US-A- 4 883 785
- VANDERHOOF J A ET AL: "TREATMENT STRATEGIES FOR SMALL BOWEL BACTERIAL OVERGROWTH IN SHORT BOWEL SYNDROME", JOURNAL OF PEDIATRIC GASTROENTEROLOGY AND NUTRITION, RAVEN PRESS, NEW YORK, NY, US, vol. 27, no. 2, 1 August 1998 (1998-08-01) , pages 155-160, XP009055963, ISSN: 0277-2116, DOI: 10.1097/00005176-199808000-00005
- anonymous: "Glenmark Initiates Phase 2 Trial for Crofelemer for Acute Infectious Diarrhea", Gelnmark Pharma , 6 December 2007 (2007-12-06), XP000002659068, Retrieved from the Internet: URL:http://www.redorbit.com/news/health/11 71136/glenmark_initiates_phase_2_trial_for _crofelemer_for_acute_infectious/index.htm l [retrieved on 2011-09-15]

## Description

### BACKGROUND

Rifaximin (INN; see The Merck Index, XIII Ed., 8304) is an antibiotic belonging to the rifamycin class of antibiotics, e.g., a pyrido-imidazo rifamycin. Rifaximin exerts its broad antibacterial activity, for example, in the gastrointestinal tract against localized gastrointestinal bacteria that cause infectious diarrhea, irritable bowel syndrome, small intestinal bacterial overgrowth, Crohn's disease, and/or pancreatic insufficiency. It has been reported that rifaximin is characterized by a negligible systemic absorption, due to its chemical and physical characteristics (Descombe J.J. et al. Pharmacokinetic study of rifaximin after oral administration in healthy volunteers. Int J Clin Pharmacol Res, 14 (2), 51-56, (1994*)).*

Rifaximin is described in Italian Patent IT 1154655 and EP 0161534. US 7,045,620 B1 discloses polymorphic forms of rifaximin.

Rifaximin is approved for the treatment of pathologies caused by non-invasive strains as Escherichia coli, micro-organism which are not able to penetrate into GI. mucosa and they remain in contact with the GI fluids.

WO 2006/102536 A2 discloses treatment of disease conditions through modulating of hydrogen sulfide produced by small intestinal bacterial overgrowth.

WO 01/11077 A2 discloses methods of diagnosing or treating irritable bowel syndrome and other disorders caused by small intestinal bacterial overgrowth.

### SUMMARY

Disclosed herein is the use of a gastrointestinal cleanser and of rifaximin for the manufacture of a medicament for preventing, ameliorating and/or treating one or more bowel diseases (BDs). In general, subjects who may benefit from the treatment with a GI cleanser and rifaximin include those who are susceptible to BDs, those who have active or acute diseases and those who are in remission from one or more BD. BDs include, for example, irritable bowel syndrome, Crohn's disease, traveler's diarrhea, ulcerative colitis, enteritis, small intestinal bacterial overgrowth or colitis. Subjects who may particularly benefit from this treatment include those who have mild to moderate IBS.

According to one aspect, provided herein is the use of a gastrointestinal (GI) cleanser and a therapeutically effective amount of rifaximin for the manufacture of a medicament for treating bowel disease (BD) comprising: administering the gastrointestinal (GI) cleanser to a subject in need thereof; and administering the therapeutically effective amount of rifaximin, wherein the administration of the gastrointestinal (GI) cleanser is within between about 1 to about 90 days before the administration of the rifaximin.

According to one embodiment, the administering of the GI cleanser and the antibiotic rifaximin results in from between about 35 - 70% of subjects with adequate relief of one or more of IBS symptoms, abdominal pain symptoms, or bloating symptoms.

According to one embodiment, the GI cleanser comprises one or more of a PEG based composition or a sodium phosphate based composition.

According to one embodiment, the GI cleanser comprises polyethylene glycol (PEG), sodium sulfate, sodium chloride, potassium chloride, and ascorbic acid.

According to one embodiment, the GI cleanser is supplied as two pouch A's comprising 100 grams of PEG 3350, 7.5 grams of sodium sulfate, 2.691 grams of sodium chloride, and 1.015 grams of potassium chloride; and two pouch B's comprising 4.7 grams of ascorbic acid, and 5.9 grams of sodium ascorbate.

According to one embodiment, the GI cleanser comprises 32 or 40 tablets comprising sodium phosphate monobasic, sodium phosphate dibasic, PEG 8000, and magnesium sterate.

According to one embodiment, the GI cleanser comprises sodium phosphate monobasic, sodium phosphate dibasic, microcrystalline cellulose, colodial silicon dioxide, and magnesium sterate.

According to one embodiment, the GI cleanser comprises Fleet® Phospho-soda® EZ-Prep; miraLAX; a bulk producing purgative; a serotonin agonist; a hyperosmotic agent; GoLytely; GlycoLax; CoLyte; or NuLytely. These purgatives are described more fully below.

According to one embodiment, the use further comprises administering an antibiotic prior to the administration of the gastrointestinal cleanser. In another embodiment, the antibiotic is administered with the GI cleanser, between the administration of the GI cleanser and the antibiotic, and/or prior to the GI cleanser. If this antibiotic is different from the antibiotic rifaximin given after the GI cleanser, then the antibiotic may also, in accordance with another embodiment, be administered with the antibiotic that is administered after the GI cleanser.

According to one embodiment, the use may further comprise administering an antibiotic with the administration of the gastrointestinal cleanser.

According to one embodiment, the use may further comprise performing a colonoscopy on the subject after the administration of the gastrointestinal cleanser.

According to the invention, the administration of the gastrointestinal cleanser is within between about 1 to about 90 days before the administration of the antibiotic rifaximin.

According to one embodiment, the administration of the gastrointestinal cleanser is within between about 1 to about 60 days; between about 1 to about 30 days; between about 1 to about 24 days; between about 1 to about 14 days; between about 1 to about 10 days; between about 1 to about 7 days; between about 1 to about 5 days; between about I to about 4 days; between about 1 to about 3 days; or between about 1 to about 2 days before the administration of the antibiotic rifaximin.

According to one embodiment, one or more of an anti-inflammatory, one or more additional antibiotics, crofelemer, or metoclopramide is to be administered to the subject. The administration of these compositions may be prior to the GI cleanser, with the GI cleanser, between the GI cleanser and the antibiotic, with the antibiotic and/or after the antibiotic.

According to one embodiment, the use may further comprise selecting subjects who respond to treatment after being treated for between about 1 and about 52 weeks or longer; and removing a responding subject from treatment wherein after removal of treatment there is a durability of response.

According to one embodiment, the subject is treated for between about 1 and about 24 weeks with the antibiotic rifaximin administered after the GI cleanser.

According to one embodiment, the bowel disease comprises, one or more of inflammatory bowel disease (IBD), Crohn's disease, enteritis, colitis, irritable bowel syndrome (IBS), travelers' diarrhea, small intestinal bacterial overgrowth, uncontrolled diarrhea-associated irritable bowel syndrome (dIBS), or diarrhea-associated irritable bowel syndrome (dIBS).

According to one embodiment, the therapeutically effective amount of the antibiotic rifaximin comprises from between about 100 mg and about 6000 mg; from between about 50 mg and about 2500 mg BID; from between about 50 mg and about 2000 mg TID; 550 mg TID; 550 mg BID; 600 mg TID; 600 mg BID; 1650 mg.

According to one embodiment, the BD comprises uncontrolled diarrhea-associated irritable bowel syndrome (dIBS).

The rifamycin class antibiotic may comprise a compound of Formula I.

According to the present invention, the rifamycin class antibiotic is rifaximin. Rifaximin may include one or more of an amorphous form, Form α, Form β, Form γ, Form δ, Form ε, Form ζ, or Form η polymorph of rifaximin.

According to one embodiment, when a subject is selected for response according to symptoms, a durability of response comprises from between about 1 and about 24 weeks of adequate relief of symptoms or from between about 1 and about 5 weeks of adequate relief of symptoms.

According to one embodiment, symptoms comprise one or more of overall BD symptoms or bloating.

According to one aspect, provided herein are uses for treating BD, comprising providing a container comprising a gastrointestinal cleanser and rifaximin, wherein the container comprises printed labeling which describes administering the gastrointestinal cleanser followed by the rifaximin; and administering the cleanser and the rifaximin from the container to the subject.

According to one aspect, provided herein are therapeutics comprising a gastrointestinal (GI) cleanser and a therapeutically effective amount of rifaximin.

According to one embodiment, the therapeutically effective amount of the antibiotic rifaximin comprises from between about 100 mg and about 6000 mg; from between about 50 mg and about 2500 mg BID; from between about 50 mg and about 2000 mg TID; 550 mg TID; 550 mg BID; 600 mg TID; 600 mg BID; 1650 mg QD; 200 mg TID; 200 mg BID or 200 mg QD.

In one embodiment, the therapeutically effective amount of the antibiotic rifaximin comprises from between about 100 mg and about 6000 mg.

In one embodiment, the therapeutically effective amount of the antibiotic rifaximin comprises 550 mg TID.

In one embodiment, the therapeutically effective amount of the antibiotic rifaximin comprises 550 mg BID.

In one embodiment, the therapeutically effective amount of the antibiotic rifaximin comprises 600 mg TID.

In one embodiment, the therapeutically effective amount of the antibiotic rifaximin comprises 600 mg BID.

In one embodiment, the therapeutically effective amount of the antibiotic rifaximin comprises 1650 mg QD.

In one embodiment, the BD comprises uncontrolled diarrhea-associated irritable bowel syndrome (dIBS). In one embodiment, the BD comprises uncontrolled constipation-associated irritable bowel syndrome (cIBS). In one embodiment, the BD comprises uncontrolled alternating irritable bowel syndrome (aIBS).

In one embodiment, symptoms comprise one or more of overall BD symptoms or bloating.

In one embodiment, adequate relief of BD symptoms comprises a reduction of BD symptoms.

In one embodiment, the reduction in BD symptoms is a reduction from baseline symptoms.

In one embodiment, baseline symptoms are established prior to treatment.

In one embodiment, adequate relief of BD symptoms comprises a 'yes' response from a subject when asked the question comprising or similar to, "In the past 7 days, have you had adequate relief of your symptom of your BD symptoms?"

In one embodiment, BD symptoms comprise one or more of cramping, pain, diarrhea, constipation, lumpy stool, watery stool, frequent stool production, abdominal pain, abdominal discomfort, and/or urgency.

In one embodiment, wherein adequate relief of bloating symptoms comprises a reduction of bloating symptoms.

In one embodiment, wherein the reduction in bloating symptoms is a reduction from baseline symptoms.

In one embodiment, baseline symptoms are established prior to treatment.

In one embodiment, adequate relief of bloating symptoms comprises a 'yes' response from a subject when asked the question comprising or similar to "In the past 7 days, have you had adequate relief of your symptom of bloating?"

In one embodiment, bloating symptoms comprise one or more of the symptoms of abdominal fullness, bloating, gas, or swelling.

In one embodiment, a BD comprises one or more of inflammatory bowel disease (IBD), Crohn's disease, enteritis, colitis, irritable bowel syndrome (IBS), travelers' diarrhea, small intestinal bacterial overgrowth, uncontrolled diarrhea-associated irritable bowel syndrome (dIBS), or diarrhea-associated irritable bowel syndrome (dIBS).

In one embodiment, the therapeutically effective amount of rifaximin comprises from between about 100 mg and about 6000 mg; 550 mg TID; 550 mg BID; 600 mg TID; 600 mg BID; or 1650 mg QD.

In one aspect, the rifamycin class antibiotic comprises one or more of an amorphous form, Form α, Form β, Form γ, Form δ, Form ε, Form ζ, Form η, Form ζ, Form η, Form α-dry, Form τ, Form β-1, Form β-2, Form ε-dry, mesylate Form or amorphous forms of rifaximin and a pharmaceutically acceptable carrier. The rifaximin may be formulated as a pharmaceutical composition.

In one embodiment, the pharmaceutical composition further comprises excipients.

According to another embodiment, the excipients are one or more of a diluting agent, binding agent, lubricating agent, disintegrating agent, coloring agent, flavorings agent or sweetening agent.

In another embodiment, the composition is formulated for selected coated and uncoated tablets, hard and soft gelatin capsules, sugar-coated pills, lozenges, wafer sheets, pellets and powders in sealed packet. In one embodiment, the composition is formulated for topical use.

According to another embodiment, the bowel related disorder is one or more of irritable bowel syndrome, travelers' diarrhea, small intestinal bacterial overgrowth, Crohn's disease or colitis.

According to one embodiment, the purgative comprises two pouch A's comprising 100 grams of polyethylene glycol (PEG) 3350, 7.5 grams of sodium sulfate, 2.691 grams of sodium chloride, and 1.015 grams of potassium chloride; and two pouch B's comprising 4.7 grams of ascorbic acid, and 5.9 grams of sodium ascorbate.

According to one embodiment, the purgative comprises 32 or 40 tablets comprising 1.102 g sodium phosphate monobasic, 0.398 g sodium phosphate dibasic, 0.1676 g PEG 8000, NF, and 0.0084 g magnesium stearate.

According to one embodiment, adequate relief of BD symptoms comprises a reduction of BD symptoms. According to one embodiment, reduction in BD symptoms is a reduction from baseline symptoms. According to one embodiment, baseline symptoms are established prior to treatment. According to one embodiment, adequate relief of BD symptoms comprise a 'yes' response from a subject when asked the question comprising or similar to, "In the past 7 days, have you had adequate relief of your symptom of your BD symptoms?" According to one embodiment, BD symptoms comprise one or more of cramping, pain, diarrhea, constipation, lumpy stool, watery stool, frequent stool production, abdominal pain, abdominal discomfort, urgency, or tenesmus. According to one embodiment, adequate relief of bloating symptoms comprises a reduction of bloating symptoms. According to one embodiment, the reduction in bloating symptoms is a reduction from baseline symptoms. According to one embodiment, baseline symptoms are established prior to treatment. According to one embodiment, adequate relief of bloating symptoms comprise a 'yes' response from a subject when asked the question comprising or is similar to, "In the past 7 days, have you had adequate relief of your symptom of bloating?" According to one embodiment, bloating symptoms comprise one or more of the symptoms of abdominal fullness, bloating, gas, or swelling.

Other embodiments of the invention are disclosed *infra.*

### DETAILED DESCRIPTION

Embodiments of the invention relate to the use of gastrointestinal cleansers and the antibiotic rifaximin to treat bowel diseases and compositions for treating bowel disease.

Rifaximin (USAN, INN; see The Merck Index, XIII Ed., 8304, CAS No. 80621-81-4), (2S,16Z,18E,20S,21S,22R, 23R,24R,25S,26S,27S,28E)-5,6,21,23,25 Pentahydroxy -27 - methoxy -2,4,11,16,20,22,24,26 - octamethyl-2,7 - (epoxypentadeca-(1,11,13) trienimino) benzofuro (4,5-e) pyrido(1,2,-a) benzimidazole-1,15(2H)-dione,25-acetate), is a semi-synthetic antibiotic produced from rifamycin O. Rifaximin is a molecule belonging to the rifamycin class of antibiotics, e.g., a pyrido-imidazo rifamycin. Rifaximin exerts a broad antibacterial activity, for example, in the gastrointestinal tract against localized gastrointestinal bacteria that cause infectious diarrhea, irritable bowel syndrome, small intestinal bacterial overgrowth, Crohn's disease, and/or pancreatic insufficiency.

Rifaximin is also described in Italian Patent IT 1154655 and EP 0161534. EP patent 0161534 discloses a process for rifaximin production using rifamycin O as the starting material (The Merck Index, XIII Ed., 8301). US 7,045,620 B 1 discloses polymorphic forms of rifaximin, as do USSN 11/658,702; USSN 61/031,329; USSN 12/119,622; USSN 12/119,630; USSN 12/119,612; USSN 12/119,600; USSN 11/873,841; Publication WO 2006/094662; and USSN 12/393012.

Rifaximin is a compound having the structure of formula II:

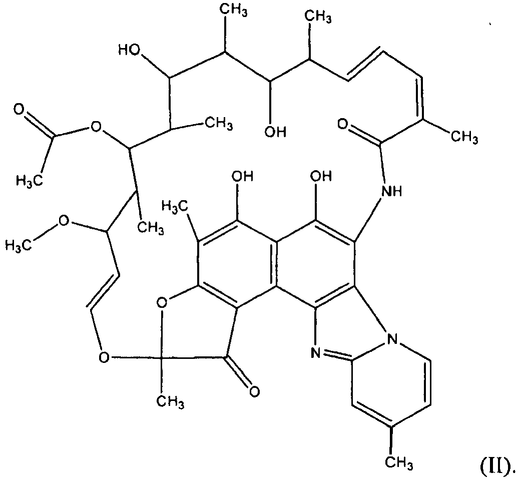

A rifamycin class antibiotic is, for example, a compound having the structure of Formula I: 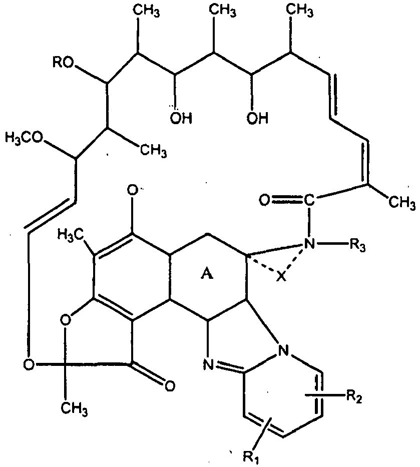 wherein A may be the structure A₁: 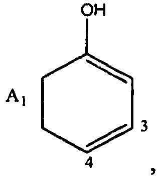 or the structure A₂ 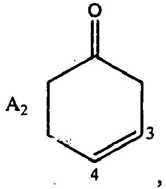 wherein, -x- is a covalent chemical bond or nil; R is hydrogen or acetyl;
R₁ and R₂ independently represent hydrogen, (C₁₋₄) alkyl, benzyloxy, mono- and di-(C₁₋₃) alkylamino-(C₁₋₄) alkyl, (C₁₋₃)alkoxy- (C₁₋₄)alkyl, hydroxymethyl, hydroxy-(C₂₋₄)-alkyl, nitro or R₁ and R₂ taken together with two consecutive carbon atoms of the pyridine nucleus form a benzene ring unsubstituted or substituted by one or two methyl or ethyl groups; R₃ is a hydrogen atom or nil; with the proviso that, when A is A₁, -x- is nil and R₃ is a hydrogen atom; with the further proviso that, when A is A₂, -x- is a covalent chemical bond and R₃ is nil.

Also described herein is a compound as defined above, wherein A is A₁ or A₂ as above indicated, -x- is a covalent chemical bond or nil, R is hydrogen or acetyl, R₁ and R₂ independently represent hydrogen, (C₁₋₄)alkyl, benzyloxy, hydroxy-(C₂₋₄) alkyl, di-(C₁₋₃) alkylamino-(C₁₋₄) alkyl, nitro or R₁ and R₂ taken together with two consecutive carbon atoms of the pyridine nucleus form a benzene ring and R₃ is a hydrogen atom or nil; with the proviso that, when A is A₁, -x- is nil and R₃ is a hydrogen atom; with the further proviso that, when A is A₂, -x- is a covalent chemical bond and R₃ is nil.

Also described herein is a compound as defined above, wherein A is A₁ or A₂ as above indicated, -x- is a covalent chemical bond or nil, R is acetyl, R₁ and R₂ independently represent hydrogen, (C₁₋₄) alkyl or R₁ and R₂ taken together with two consecutive carbon atoms of the pyridine nucleus form a benzene ring and R₃ is a hydrogen atom or nil; with the proviso that, when A is A₁, - x- is nil and R₃ is a hydrogen atom; with the further proviso that, when A is A₂, -x- is a covalent chemical bond and R₃ is nil.

Also described herein is a compound as defined above, which is 4-deoxy-4'-methyl-pyrido[1',2'-1,2]imidazo [5,4-c]rifamycin SV. Also described herein is a compound as defined above, which is 4-deoxy-pyrido [1',2':1,2]imidazo [5,4-c] rifamycin SV.

Also described herein is a compound as defined above, wherien A is as described above,-x- is a covalent chemical bond or nil; R is hydrogen or acetyl; R₁ and R₂ independently represent hydrogen, (C₁₋₄) alkyl, benzyloxy, mono- and di-(C₁₋₃)alkylamino(C₁₋₄)alkyl, (C₁₋₃)alkoxy- (C₁₋₄)alkyl, hydroxymethyl, hydroxy-(C₂₋₄)-alkyl, nitro or R₁ and R₂ taken together with two consecutive carbon atoms of the pyridine nucleus form a benzene ring unsubstituted or substituted by one or two methyl or ethyl groups; R₃ is a hydrogen atom or nil; with the proviso that, when A is A₁, -x- is nil and R₃ is a hydrogen atom; with the further proviso that, when A is A₂, -x- is a covalent chemical bond and R₃ is nil.

As used herein, "bowel disease" include, for example, one or more of inflammatory bowel disease (IBD), Crohn's disease, enteritis, colitis, irritable bowel syndrome (IBS), travelers' diarrhea, small intestinal bacterial overgrowth, uncontrolled diarrhea-associated irritable bowel syndrome (dIBS), constipation predominant IBS or alternating IBS.

### Treatment

Provided herein, according to one aspect, are uses for the manufacture of a medicament for treating and/or preventing bowel disease (BD). In one embodiment, a gastrointestinal (GI) cleanser is administered to a subject at risk of or suffering from a bowel disease. GI cleansers, for example, include purgatives and constipation relievers as described herein and as known to one of skill in the art. Following the administration of the GI cleanser, a therapeutically effective amount of rifaximin is administered to the subject, wherein the administration of the GI cleanser is within between about 1 to about 90 days before the administration of the rifaximin. In one embodiment, the administration of a further antibiotic is begun with the GI cleanser and is continued thereafter for a period of time. In other embodiments a further antibiotic is given prior to the administration of the GI cleanser, and in yet another embodiment, a further antibiotic is given prior to and during the administration of the GI cleanser.

In certain embodiments, the further antibiotic comprises one or more of a rifamycin, aminoglycoside, amphenicol, ansamycin, β-Lactam, carbapenem, cephalosporin, cephamycin, monobactam, oxacephem, lincosamide, macrolide, polypeptide, tetracycline, or a 2,4-diaminopyrimidine class antibiotic. Exemplary antibiotics of these classes are listed below.

In certain embodiments, the administering of the gastrointestinal cleanser and the antibiotic rifaximin results in from between about 35 - 70% of subjects with or experiencing adequate relief of one or more of IBS symptoms, abdominal pain symptoms, or bloating symptoms.

In certain embodiments, it may be advantageous to perform a colonoscopy on the subject after the administration of the gastrointestinal cleanser. A colonoscopy allows a visual inspection of the colon and in some instances, allows for diagnosis of underlying symptoms or confirmation of diagnosis. Without wishing to be bound by any particular scientific theory, a colonoscopy may be beneficial to treatment by causing the muscles of the colon to contract.

The administration of the gastrointestinal cleanser is within between about 1 to about 90 days before the administration of the antibiotic rifaximin. In other embodiments, the administration of the gastrointestinal cleanser is within between about 1 to about 60 days; between about 1 to about 30 days; between about 1 to about 24 days; between about 1 to about 14 days; between about 1 to about 10 days; between about 1 to about 7 days; between about 1 to about 5 days; between about 1 to about 4 days; between about 1 to about 3 days; or between about 1 to about 2 days before the administration of the antibiotic rifaximin. It may be advantageous in some circumstances to begin antibiotic therapy prior to the cleanser administration and/or administer an antibiotic during administration of the cleanser. Antibiotic give prior to or with a cleanser may be an antibiotic that is the same as or different from the antibiotic rifaximin given after the cleanser. If the antibiotic is the same as that given after the cleanser it may be the same or a higher or lower dose and it may be administered in a different form (oral, topical, rectal, etc) and/or dosing regime.

In certain embodiments, it may be advantageous to co-administer other therapeutics with the cleanser and/or the antibiotic. Such co-administered therapeutics include, for example, one or more of an anti-inflammatory, one or more additional antibiotics, an anti emetic, an anti- diarrheal, crofelemer, or metoclopramide.

In certain embodiments, the uses described herein may further comprise selecting subjects who respond to treatment after being treated for between about 1 and about 52 weeks or longer; and removing a responding subject from treatment wherein after removal of treatment there is a durability of response. Subjects, may, for example, be treated for between about 1 and about 24 weeks. Methods relating to the durability of response are fully described in US Application No. 61/031679.

In certain embodiments, the therapeutically effective amount of the antibiotic rifaximin administered after the administration of the cleanser will be between about 50 mg and about 6000 mg; from between about 50 mg and about 3000 mg BID; from between about 50 mg and about 2000 mg TID; 550 mg TID; 550 mg BID; 600 mg TID; 600 mg BID; 1650 mg QD; 200 mg TID, 200 mg BID, or 200 mg QD. These doses are also appropriate for an antibiotic given prior to or during the administration of the cleanser.

According to one aspect, uses for treating BD are provided, which comprise providing a container comprising a gastrointestinal cleanser and rifaximin, wherein the container comprises printed labeling which describes administering the gastrointestinal cleanser followed by the rifaximin; and administering the cleanser and the rifaximin from the container to the subject. The GI cleanser may be one descried herein or a combination thereof. It may also be one known to one of skill in the art to be effective. One of skill in the art, having the benefit of this disclosure would know what would be considered effective.

### GI Cleansers

GI cleansers, as used herein include purgatives and constipation relievers, which are also known as, oral laxative solutions (e.g., laxative preparations), colon clearing composition, bowel irrigation, enemas, rectal pulsed irrigation and bowel preparations. As used herein, GI cleansers also refer to compounds or compositions that free the bowel from solid matter (e.g., stool). Combinations of GI cleansers and other stimulation compositions may be useful, for example, use of a stimulant laxative (e.g., bisacodyl) in combination with an osmotic laxative. The GI cleanser may be one or more of a PEG based composition or a sodium phosphate based composition as further described below. GI cleansers may also be combinations of the below described cleansers or other cleansers known by one of skill in the art to be effective according to the methods described herein.

Exemplary stimulant laxatives include, for example, Aloe, 250-1000 mg; Bisacodyl, about 5-80 mg; Casanthranol, 30 to 360 mg; Cascara aromatic fluid extract, 2-24 ml; Cascara sagrada bark, 300-4000 mg; Cascada sagrada extract, 300 to 2000 mg; Cascara sagrada fliuid extract, 0.5 to 5 ml; Castor oil, 15-240 ml.; Danthron, 75-300 mg; Dehydrocholic Acid, 250-2000 mg; Phenolphthalein, 30-1000 mg; Sennosides A and B, 12-200 mg; and Picosulfate, 1-100 mg. Larger or smaller doses may be used, as necessary, to produce a bowel movement within less than about 12 hours, while avoiding unnecessary discomfort.

Bisacodyl is a stimulant laxative, available without prescription, used to treat constipation. Bisacodyl is available in tablets, suppositories, and in premixed enema formulations. Bisacodyl enemas are usually effective to produce a bowel movement in about 20 minutes, suppositories usually produce a bowel movement in about an hour, and oral administration of a tablet usually results in a bowel movement in about 3 to 6 hours. As shown in U.S. Pat. No. 5,710,183, Polyethylene Glycol (PEG) 3350 has been used alone as a medication to treat constipation by improving bowel motility, stool formation, or both. PEG has also been combined with soluble fiber to make a safe and effective laxative, as also shown in U.S. Pat. No. 5,710,183, and PEG can be combined with soluble fiber to improve bowel function. Exemplary doses of PEG to treat constipation include 17 to 34 grams of PEG daily. Higher doses of PEG can be used to produce one or two bowel movements within 24 hours without causing profuse diarrhea. In one example, a package comprises 2 L of NuLYTELY with 4 Bisacodyl Tablets 20 mg (5 mg each) attached to the outside of the 2-liter jug. Each dose of the NuLYTELY solution contained: Polyethylene Glycol 3350, NF, 210 g., Sodium Chloride, USP 5.60 g., Sodium Bicarbonate, USP 2.86 grams, Potassium Chloride, USP 0.74 grams, and optionally, 1 gram of a flavor ingredient in water to make 2 L. PEG has also been shown to be effective as a colonic purgative when large amounts of PEG are administered in large volumes of a dilute salt solution. Usually about 250 to about 400 grams of PEG are administered to the patient in about 4 liters of an electrolyte solution in water. Oral administration of PEG can be used to produce an overnight bowel movement.

Exemplary PEG based solutions comprise, for example, polyethylene glycol (PEG), sodium sulfate, sodium chloride, potassium chloride, ascorbic acid; or PEG, sodium sulfate, sodium chloride, potassium chloride, ascorbic acid, and sodium ascorbate; or PEG 3350, sodium sulfate, sodium chloride, potassium chloride, ascorbic acid, and sodium ascorbate. In one embodiment, the PEG purgative is supplied as two pouch A's comprising 100 grams of polyethylene glycol (PEG) 3350, 7.5 grams of sodium sulfate, 2.691 grams of sodium chloride, and 1.015 grams of potassium chloride; and two pouch B's comprising 4.7 grams of ascorbic acid, and 5.9 grams of sodium ascorbate. It is well know by one of skill in the art how to administer such compositions to produce cleansing.

A method of cleansing the colon of a mammal useful in the uses detailed herein, comprises, for example, administering orally to a subject a cleansing fluid comprising, per liter, the following components, a) 80 to 350 g polyethylene glycol; b) 3 to 20 g of a mixture of ascorbic acid and one or more salts of ascorbic acid; c) 1 to 15 g of an alkali metal or alkaline earth metal sulphate or a mixture of alkali metal or alkaline earth metal sulphates; and d) optionally one or more electrolytes selected from sodium chloride, potassium chloride and sodium hydrogen carbonate, the volume of fluid administered being from 1.5 to 3 litres for an adult human and pro rata for a mammal other than an adult human.

In certain embodiments, a sodium phosphate GI cleanser useful in the uses described herein comprises 32 or 40 tablets comprising sodium phosphate monobasic, sodium phosphate dibasic, PEG 8000, and magnesium sterate. Another example, comprises sodium phosphate monobasic, sodium phosphate dibasic, microcrystalline cellulose, colodial silicon dioxide, and magnesium sterate. Other useful GI cleansers include, for example, Fleet® Phospho-soda® EZ-PrepTM; miraLAX; a bulk producing purgative; a serotonin agonist; a hyperosmotic agent; GoLytely; GlycoLax; CoLyte; or NuLytely. One of skill in the art would know how to administer each of these compositions.

Other GI cleansers useful in the uses and formulations (e.g., kits) described herein, include, for example, those described by Fordtran et al. (WO87/00754), including the reduced sodium sulphate solution (RSS). This solution comprises no sodium sulphate but instead has a relatively high concentration of polyethylene glycol (75 to 300 g/l). A solution disclosed in WO87/00754 comprises PEG 3350 (120 g/l), sodium bicarbonate (1.68 g/l), potassium chloride (0.74 g/l) and sodium chloride (1.46 g/l) and it is also administered in a quantity of 4 litres. Another exemplary solution is commercialized by Braintree Laboratories Inc (Braintree, Mass., U.S.A.) under the name NuLYTELY.RTM. (initially also under the name GoLYTELY-RSS). The NuLYTELY composition comprises PEG 3350 (105 g/l), sodium bicarbonate (1.43 g/l), potassium chloride (0.37 g/l) and sodium chloride (2.80 g/l) and it is supplied in dry powder form for making up to 4 liters. WO 89/05659 (Borody) describes yet another exemplary GI cleanser useful in the uses and formulations described herein. This is an orthostatic lavage solution comprising polyethylene glycol, electrolytes and from 0.25 to 50 g/l ascorbic acid (vitamin C) or a salt thereof.

Other GI cleansers useful in the uses and formulations described herein, include, for example, bulk producing purgatives (psyllium husk (Metamucil), methylcellulose (Citrucel), polycarbophil, dietary fiber, apples); Serotonin agonist (e.g., Tegaserod); hyperosmotic agents (e.g., glycerin suppositories, sorbitol, lactulose, and polyethylene glycol (PEG)). Brand names for these solutions include GoLytely (a white powder in a 4 liter jug for reconstitution, containing 236g polyethylene glycol 3350, 22.74g sodium sulfate (anhydrous), 6.74g sodium bicarbonate, 5.86g sodium chloride and 2.97g potassium chloride. When dissolved in water to a volume of 4 liters, GoLYTELY (PEG-3350 and electrolytes for oral solution) is an isosmotic solution having a mildly salty taste. GoLYTELY may be, for example, administered orally or via nasogastric tube as a gastrointestinal lavage).

OsmoPrep comprises 48 grams of sodium phosphate (32 tablets), induces diarrhea, which effectively cleanses the entire colon. Each administration has a purgative effect for approximately 1 to 3 hours. The primary mode of action is thought to be through the osmotic effect of sodium, causing large amounts of water to be drawn into the colon, promoting evacuation. Each OsmoPrep tablet contains 1.102 grams of sodium phosphate monobasic monohydrate, USP and 0.398 grams of sodium phosphate dibasic anhydrous, USP for a total of 1.5 grams of sodium phosphate per tablet. Inert ingredients include polyethylene glycol 8000, N.F; and magnesium stearate, NF. OsmoPrep is gluten-free. The recommended dose of OsmoPrep Tablets for colon cleansing for adult patients is 32 tablets (48 grams of sodium phosphate) taken orally with a total of 2 quarts of clear liquids in the following manner: the evening before the colonoscopy procedure: Take 4 OsmoPrep Tablets with 8 ounces of clear liquids every 15 minutes for a total of 20 tablets. On the day of the colonoscopy procedure: Starting 3-5 hours before the procedure, take 4 OsmoPrep Tablets with 8 ounces of clear liquids every 15 minutes for a total of 12 tablets. Patients should be advised of the importance of taking the recommended fluid regimen. It is recommended that patients receiving OsmoPrep be advised to adequately hydrate before, during, and after the use of OsmoPrep. Patients should not use OsmoPrep for colon cleansing within seven days of previous administration. No additional enema or laxative is required, and patients should be advised NOT to take additional agents, particularly those containing sodium phosphate.

VisicolP® (sodium phosphate monobasic monohydrate, USP, and sodium phosphate dibasic anhydrous, USP) is a purgative used to clean the colon prior to colonoscopy. Each tablet contains 1.102 grams of sodium phosphate monobasic monohydrate, USP and 0.398 grams of sodium phosphate dibasic anhydrous, USP for a total of 1.5 grams of sodium phosphate per tablet. Inert ingredients include microcrystalline cellulose (MCC), NF; magnesium stearate, NF; and colloidal silicon dioxide, NF. VisicolP® is gluten-free. VisicolP^{®} tablets, taken in two doses of 30 grams (the complete regimen contains a total of 60 grams of sodium phosphate) approximately twelve hours apart, induces diarrhea, which effectively cleanses the entire colon. Each administration has a purgative effect for approximately 1 to 3 hours. The primary mode of action is thought to be through osmotic action of sodium, causing large amounts of water to be drawn into the colon, promoting colon evacuation. The recommended dose of Visicol® Tablets for colon cleansing for adult patients is 40 tablets (60 grams of sodium phosphate) taken orally with a total of 3.6 quarts of clear liquids in the following manner:

The evening before the colonoscopy procedure: Take 3 Visicol® Tablets (the last dose will be 2 Visicol ® Tablets) with 8 ounces of clear liquids every 15 minutes for a total of 20 tablets. On the day of the colonoscopy procedure: Starting 3-5 hours before the procedure, take 3 Visicol® Tablets (the last dose will be 2 Visicol® Tablets) with 8 ounces of clear liquids every 15 minutes for a total of 20 tablets. It is recommended that patients receiving Visicol® be advised to adequately hydrate before, during, and after the use of Visicol®. Patients should not use Visico® within seven days of previous administration. No additional enema or laxative is required, and patients should be advised NOT to take additional agents, particularly those containing sodium phosphate.

Other exemplary GI cleansers include those detailed in Tables 1 and 2:

**Table 1:**

| | **Grams/Tablet** | **% by weight** |
|---|---|---|
| | | |
| Sodium Phosphate Salt: | | |
| Monobasic | 1.102 | 65.752 |
| Dibasic | 0.398 | 23.747 |
| Inert: | | |
| PEG 8000, NF | 0.1676 | 10.000 |
| Magnesium Sterate, NF | 0.0084 | 0.502 |
| Total | 1.6760 | 100.001 |

**Table 2:**

| **Ingredients** | **Grams /tablet** | **%by wt** |
|---|---|---|
| **Sodium Phosphate Salts:** | | |
| Monobasic | 1.102 | 62.436 |
| Dibasic | 0.398 | 22.550 |
| **Inert ingredients:** | | |
| Microcrystalline cellulose | 0.22950 | 13.003 |
| Magnesium stearate | 0.02645 | 1.499 |
| Colodial silicone dioxide | 0.00885 | .501 |
| | 1.765 | 99.989 |

GI cleansers that are provided as dry powders or concentrated liquids may be, for example, stirred and dissolve in any a beverage (cold, hot or room temperature) and then administered (e.g., taken orally). GI cleansers provided as liquids may be administered.

In certain embodiments, other therapeutic agents may be co-administered with the GI cleanser or with the antibiotic or both. These other therapeutic agent(s) may also be given prior to the GI cleanser, during the GI cleanser or between administration of the GI cleanser and the antibiotic.

### Antibiotics

Antibiotics include, for example, aminoglycosides, such as amikacin, apramycin, arbekacin, bambermycins, butirosin, dibekacin, dihydrostreptomycin, fortimicin(s), fradiomycin, gentamicin, ispamicin, kanamycin, micronomicin, neomycin, neomycin undecylenate, netilmicin, paromomycin, ribostamycin, sisomicin, spectinomycin, streptomycin, streptonicozid, and tobramycin; amphenicols, such as azidamfenicol, chloramphenicol, chloramphenicol palmirate, chloramphenicol pantothenate, florfenicol, and thiamphenicol; ansamycins, such as rifampin, rifabutin, rifapentine, and rifaximin; .beta.-Lactams, such as amidinocillin, amdinocillin, pivoxil, amoxicillin, ampicillin, aspoxicillin, azidocillin, azlocillin, bacampicillin, benzylpenicillinic acid, benzylpenicillin, carbenicillin, carfecillin, carindacillin, clometocillin, cloxacillin, cyclacillin, dicloxacillin, diphenicillin, epicillin, fenbenicillin, floxicillin, hetacillin, lenampicillin, metampicillin, methicillin, mezlocillin, nafcillin, oxacillin, penamecillin, penethamate hydriodide, penicillin G benethamine, penicillin G benzathine, penicillin G benzhydrylamine, penicillin G calcium, penicillin G hydragamine, penicillin G potassium, penicillin G, procaine, penicillin N, penicillin O, penicillin V, penicillin V benzathine, penicillin V hydrabamine, penimepicycline, phenethicillin, piperacillin, pivapicillin, propicillin, quinacillin, sulbenicillin, talampicillin, temocillin and ticarcillin; carbapenems, such as imipenem; cephalosporins, such as 1-carba (dethia) cephalosporin, cefactor, cefadroxil, cefamandole, cefatrizine, cefazedone, cefazolin, cefixime, cefmenoxime, cefodizime, cefonicid, cefoperazone, ceforanide, cefotaxime, cefotiam, cefpimizole, cefpirimide, cefpodoxime proxetil, cefroxadine, cefsulodin, ceftazidime, cefteram, ceftezole, ceftibuten, ceftizoxime, ceftriaxone, cefuroxime, cef*uizonam, cephacetrile sodium, cephalexin, cephaloglycin, cephaloridine, cephalosporin, cephalothin, cephapirin sodium, cephradine, pivcefalexin, cephalothin, cefaclor, cefotetan, cefprozil, loracarbef, cefetamet, and cefepime; cephamycins such as cefbuperazone, cefmetazole, cefminox, cefetan, and cefoxitin; monobactams such as aztreonam, carumonam, and tigemonan; oxacephems such as flomoxef and moxolactam; lincosamides such as clindamycin and lincomycin; macrolides such as azithromycin, carbomycin, clarithromycin, erythromycin(s) and derivatives, josamycin, leucomycins, midecamycins, miokamycin, oleandomycin, primycin, rokitamycin, rosaramicin, roxithromycin, spiramycin and troleandomycin; polypeptides such as amphomycin, bacitracin, capreomycin, colistin, enduracidin, enylomycin, fusafungine, gramicidin(s), gramicidin S, mikamycin, polymyxin, polymyxin ..beta.-methanesulfonic acid, pristinamycin, ristocetin, teicoplanin, thiostrepton, tuberactinomycin, tyrocidine, tyrothricin, vancomycin, viomycin(s), virginiamycin and zinc bacitracin; tetracyclines such as spicycline, chlortetracycline, clomocycline, demeclocycline, doxycycline, guamecycline, lymecycline, meclocycline, methacycline, minocycline, oxytetracycline, penimepicycline, pipacycline, rolitetracycline, sancycline, senociclin and tetracycline; and 2,4-diaminopyrimidines such as brodimoprim, tetroxoprim and trimethoprim; nitrofurans such as furaltadone, furazolium, nifuradene, nifuratel, nifurfoline, nifurpirinol, nifurprazine, nifurtoinol and nitrofurantoin; quinolones such as amifloxacin, cinoxacin, ciprofloxacin, difloxacin, enoxacin, fleroxacin, flumequine, lomefloxacin, miloxacin, nalidixic acid, norfloxacin, ofloxacin, oxolinic acid, perfloxacin, pipemidic acid, piromidic acid, rosoxacin, temafloxacin, and tosufloxacin; sulfonamides such as acetyl sulfamethoxypyrazine, acetyl sulfisoxazole, azosulfamide, benzylsulfamide, chloramine-.beta., chloramine-T, dichloramine-T, formosulfathiazole, N.sub.2-formyl-sulfisomidine, N.sub.4-.beta.-D-glucosylsulfanilamide, mafenide, 4'-(methyl-sulfamoyl)sulfanilanilide, p-nitrosulfathiazole, noprylsulfamide, phthalylsulfacetamide, phthalylsulfathiazole, salazosulfadimidine, succinylsulfathiazole, sulfabenzamide, sulfacetamide, sulfachlorpyridazine, sulfachrysoidine, sulfacytine, sulfadiazine, sulfadicramide, sulfadimethoxine, sulfadoxine, sulfaethidole, sulfaguanidine, sulfaguanol, sulfalene, sulfaloxic acid, sulfamerazine, sulfameter, sulfamethazine, sulfamethizole, sulfamethomidine, sulfamethoxazole, sulfamethoxypyridazine, sulfametrole, sulfamidochrysoidine, sulfamoxole, sulfanilamide, sulfanilamidomethanesulfonic acid triethanolamine salt, 4-sulfanilamidosalicyclic acid, N.sub.4-sulfanilylsulfanilamide, sulfanilylurea, N-sulfanilyl-3,4-xylamide, sulfanitran, sulfaperine, sulfaphenazole, sulfaproxyline, sulfapyrazine, sulfapyridine, sulfasomizole, sulfasymazine, sulfathiazole, sulfathiourea, sulfatolamide, sulfisomidine and sulfisoxazole; sulfones, such as acedapsone, acediasulfone, acetosulfone, dapsone, diathymosulfone, glucosulfone, solasulfone, succisulfone, sulfanilic acid, p-sulfanilylbenzylamine, p,p'-sulfonyldianiline-N,N' digalactoside, sulfoxone and thiazolsulfone; lipopeptides such as daptomycin; oxazolidones such as linezolid; ketolides such as telithromycin; and miscellaneous antibiotics such as clofoctol, hexedine, magainins, methenamine, methenamine anhydromethylene-citrate, methenamine hippurate, methenamine mandelate, methenamine sulfosalicylate, nitroxoline, squalamine, xibornol, cycloserine, mupirocin, and tuberin.

Examples of antimicrobial and antibiotic agents that are suitable for use include, without limitation, mandelic acid, 2,4-dichlorobenzenemethanol, 4-[bis(ethylthio)methyl]-2-methoxyphenol, 4-epi-tetracycline, 4-hexylresorcinol, 5,12-dihydro-5,7,12,14-tetrazapentacen, 5-chlorocarvacrol, 8-hydroxyquinoline, acetarsol, acetylkitasamycin, acriflavin, alatrofloxacin, ambazon, amfomycin, amikacin, amikacin sulfate, aminoacridine, aminosalicylate calcium, aminosalicylate sodium, aminosalicylic acid, ammoniumsulfobituminat, amorolfin, amoxicillin, amoxicillin sodium, amoxicillin trihydrate, amoxicillin-potassium clavulanate combination, amphotericin B, ampicillin, ampicillin sodium, ampicillin trihydrate, ampicillin-sulbactam, apalcillin, arbekacin, aspoxicillin, astromicin, astromicin sulfate, azanidazole, azidamfenicol, azidocillin, azithromycin, azlocillin, aztreonam, bacampicillin, bacitracin, bacitracin zinc, bekanamycin, benzalkonium, benzethonium chloride, benzoxonium chloride, berberine hydrochloride, biapenem, bibrocathol, biclotymol, bifonazole, bismuth subsalicylate, bleomycin antibiotic complex, bleomycin hydrochloride, bleomycin sulfate, brodimoprim, bromochlorosalicylanilide, bronopol, broxyquinolin, butenafine, butenafine hydrochloride, butoconazol, calcium undecylenate, candicidin antibiotic complex, capreomycin, carbenicillin, carbenicillin disodium, carfecillin, carindacillin, carumonam, carzinophilin, caspofungin acetate, cefacetril, cefaclor, cefadroxil, cefalexin, cefalexin hydrochloride, cefalexin sodium, cefaloglycin, cefaloridine, cefalotin, cefalotin sodium, cefamandole, cefamandole nafate, cefamandole sodium, cefapirin, cefapirin sodium, cefatrizine, cefatrizine propylene glycol, cefazedone, cefazedone sodium salt, cefazolin, cefazolin sodium, cefbuperazone, cefbuperazone sodium, cefcapene, cefcapene pivoxil hydrochloride, cefdinir, cefditoren, cefditoren pivoxil, cefepime, cefepime hydrochloride, cefetamet, cefetamet pivoxil, cefixime, cefinenoxime, cefinetazole, cefinetazole sodium, cefininox, cefininox sodium, cefmolexin, cefodizime, cefodizime sodium, cefonicid, cefonicid sodium, cefoperazone, cefoperazone sodium, ceforanide, cefoselis sulfate, cefotaxime, cefotaxime sodium, cefotetan, cefotetan disodium, cefotiam, cefotiam hexetil hydrochloride, cefotiam hydrochloride, cefoxitin, cefoxitin sodium, cefozopran hydrochloride, cefpiramide, cefpiramide sodium, cefpirome, cefpirome sulfate, cefpodoxime, cefpodoxime proxetil, cefprozil, cefquinome, cefradine, cefroxadine, cefsulodin, ceftazidime, cefteram, cefteram pivoxil, ceftezole, ceftibuten, ceftizoxime, ceftizoxime sodium, ceftriaxone, ceftriaxone sodium, cefuroxime, cefuroxime axetil, cefuroxime sodium, cetalkonium chloride, cetrimide, cetrimonium, cetylpyridinium, chloramine T, chloramphenicol, chloramphenicol palmitate, chloramphenicol succinate sodium, chlorhexidine, chlormidazole, chlormidazole hydrochloride, chloroxylenol, chlorphenesin, chlorquinaldol, chlortetracycline, chlortetracycline hydrochloride, ciclacillin, ciclopirox, cinoxacin, ciprofloxacin, ciprofloxacin hydrochloride, citric acid, clarithromycin, clavulanate potassium, clavulanate sodium, clavulanic acid, clindamycin, clindamycin hydrochloride, clindamycin palmitate hydrochloride, clindamycin phosphate, clioquinol, cloconazole, cloconazole monohydrochloride, clofazimine, clofoctol, clometocillin, clomocycline, clotrimazol, cloxacillin, cloxacillin sodium, colistin, colistin sodium methanesulfonate, colistin sulfate, cycloserine, dactinomycin, danofloxacin, dapsone, daptomycin, daunorubicin, DDT, demeclocycline, demeclocycline hydrochloride, dequalinium, dibekacin, dibekacin sulfate, dibrompropamidine, dichlorophene, dicloxacillin, dicloxacillin sodium, didecyldimethylammonium chloride, dihydrostreptomycin, dihydrostreptomycin sulfate, diiodohydroxyquinolin, dimetridazole, dipyrithione, dirithromycin, DL-menthol, D-menthol, dodecyltriphenylphosphonium bromide, doxorubicin, doxorubicin hydrochloride, doxycycline, doxycycline hydrochloride, econazole, econazole nitrate, enilconazole, enoxacin, enrofloxacin, eosine, epicillin, ertapenem sodium, erythromycin, erythromycin estolate, erythromycin ethyl succinate, erythromycin lactobionate, erythromycin stearate, ethacridine, ethacridine lactate, ethambutol, ethanoic acid, ethionamide, ethyl alcohol, eugenol, exalamide, faropenem, fenticonazole, fenticonazole nitrate, fezatione, fleroxacin, flomoxef, flomoxef sodium, florfenicol, flucloxacillin, flucloxacillin magnesium, flucloxacillin sodium, fluconazole, flucytosine, flumequine, flurithromycin, flutrimazole, fosfomycin, fosfomycin calcium, fosfomycin sodium, framycetin, framycetin sulphate, furagin, furazolidone, fusafungin, fusidic acid, fusidic acid sodium salt, gatifloxacin, gemifloxacin, gentamicin antibiotic complex, gentamicin cla, gentamycin sulfate, glutaraldehyde, gramicidin, grepafloxacin, griseofulvin, halazon, haloprogine, hetacillin, hetacillin potassium, hexachlorophene, hexamidine, hexetidine, hydrargaphene, hydroquinone, hygromycin, imipenem, isepamicin, isepamicin sulfate, isoconazole, isoconazole nitrate, isoniazid, isopropanol, itraconazole, josamycin, josamycin propionate, kanamycin, kanamycin sulphate, ketoconazole, kitasamycin, lactic acid, lanoconazole, lenampicillin, leucomycin A1, leucomycin A13, leucomycin A4, leucomycin A5, leucomycin A6, leucomycin A7, leucomycin A8, leucomycin A9, levofloxacin, lincomycin, lincomycin hydrochloride, linezolid, liranaftate, 1-menthol, lomefloxacin, lomefloxacin hydrochloride, loracarbef, lymecyclin, lysozyme, mafenide acetate, magnesium monoperoxophthalate hexahydrate, mecetronium ethylsulfate, mecillinam, meclocycline, meclocycline sulfosalicylate, mepartricin, merbromin, meropenem, metalkonium chloride, metampicillin, methacycline, methenamin, methyl salicylate, methylbenzethonium chloride, methylrosanilinium chloride, meticillin, meticillin sodium, metronidazole, metronidazole benzoate, mezlocillin, mezlocillin sodium, miconazole, miconazole nitrate, micronomicin, micronomicin sulfate, midecamycin, minocycline, minocycline hydrochloride, miocamycin, miristalkonium chloride, mitomycin c, monensin, monensin sodium, morinamide, moxalactam, moxalactam disodium, moxifloxacin, mupirocin, mupirocin calcium, nadifloxacin, nafcillin, nafcillin sodium, naftifine, nalidixic acid, natamycin, neomycin a, neomycin antibiotic complex, neomycin C, neomycin sulfate, neticonazole, netilmicin, netilmicin sulfate, nifuratel, nifuroxazide, nifurtoinol, nifurzide, nimorazole, niridazole, nitrofurantoin, nitrofurazone, nitroxolin, norfloxacin, novobiocin, nystatin antibiotic complex, octenidine, ofloxacin, oleandomycin, omoconazol, orbifloxacin, ornidazole, ortho-phenylphenol, oxacillin, oxacillin sodium, oxiconazole, oxiconazole nitrate, oxoferin, oxolinic acid, oxychlorosene, oxytetracycline, oxytetracycline calcium, oxytetracycline hydrochloride, panipenem, paromomycin, paromomycin sulfate, pazufloxacine, pefloxacin, pefloxacin mesylate, penamecillin, penicillin G, penicillin G potassium, penicillin G sodium, penicillin V, penicillin V calcium, penicillin V potassium, pentamidine, pentamidine diisetionate, pentamidine mesilas, pentamycin, phenethicillin, phenol, phenoxyethanol, phenylmercuriborat, PHMB, phthalylsulfathiazole, picloxydin, pipemidic acid, piperacillin, piperacillin sodium, pipercillin sodium-tazobactam sodium, piromidic acid, pivampicillin, pivcefalexin, pivmecillinam, pivmecillinam hydrochloride, policresulen, polymyxin antibiotic complex, polymyxin B, polymyxin B sulfate, polymyxin B 1, polynoxylin, povidone-iodine, propamidin, propenidazole, propicillin, propicillin potassium, propionic acid, prothionamide, protiofate, pyrazinamide, pyrimethamine, pyrithion, pyrroInitrin, quinoline, quinupristin-dalfopristin, resorcinol, ribostamycin, ribostamycin sulfate, rifabutin, rifampicin, rifamycin, rifapentine, rifaximin, ritiometan, rokitamycin, rolitetracycline, rosoxacin, roxithromycin, rufloxacin, salicylic acid, secnidazol, selenium disulphide, sertaconazole, sertaconazole nitrate, siccanin, sisomicin, sisomicin sulfate, sodium thiosulfate, sparfloxacin, spectinomycin, spectinomycin hydrochloride, spiramycin antibiotic complex, spiramycin b, streptomycin, streptomycin sulphate, succinylsulfathiazole, sulbactam, sulbactam sodium, sulbenicillin disodium, sulbentin, sulconazole, sulconazole nitrate, sulfabenzamide, sulfacarbamide, sulfacetamide, sulfacetamide sodium, sulfachlorpyridazine, sulfadiazine, sulfadiazine silver, sulfadiazine sodium, sulfadicramide, sulfadimethoxine, sulfadoxine, sulfaguanidine, sulfalene, sulfamazone, sulfamerazine, sulfamethazine, sulfamethazine sodium, sulfamethizole, sulfamethoxazole, sulfamethoxazol-trimethoprim, sulfamethoxypyridazine, sulfamonomethoxine, sulfamoxol, sulfanilamide, sulfaperine, sulfaphenazol, sulfapyridine, sulfaquinoxaline, sulfasuccinamide, sulfathiazole, sulfathiourea, sulfatolamide, sulfatriazin, sulfisomidine, sulfisoxazole, sulfisoxazole acetyl, sulfonamides, sultamicillin, sultamicillin tosilate, tacrolimus, talampicillin hydrochloride, teicoplanin A2 complex, teicoplanin A2-1, teicoplanin A2-2, teicoplanin A2-3, teicoplanin A2-4, teicoplanin A2-5, teicoplanin A3, teicoplanin antibiotic complex, telithromycin, temafloxacin, temocillin, tenoic acid, terbinafine, terconazole, terizidone, tetracycline, tetracycline hydrochloride, tetracycline metaphosphate, tetramethylthiuram monosulfide, tetroxoprim, thiabendazole, thiamphenicol, thiaphenicol glycinate hydrochloride, thiomersal, thiram, thymol, tibezonium iodide, ticarcillin, ticarcillin-clavulanic acid mixture, ticarcillin disodium, ticarcillin monosodium, tilbroquinol, tilmicosin, timidazole, tioconazole, tobramycin, tobramycin sulfate, tolciclate, tolindate, tolnaftate, toloconium metilsulfat, toltrazuril, tosufloxacin, triclocarban, triclosan, trimethoprim, trimethoprim sulfate, triphenylstibinsulfide, troleandomycin, trovafloxacin, tylosin, tyrothricin, undecoylium chloride, undecylenic acid, vancomycin, vancomycin hydrochloride, viomycin, virginiamycin antibiotic complex, voriconazol, xantocillin, xibomol and zinc undecylenate.

Particularly suitable antibiotics for use described herein include, for example neomycin, metronidazole, teicoplanin, doxycycline, tetracycline, ciprofloxacin, augmentin, cephalexin (e.g., Keflex), penicillin, ampicillin, kanamycin, rifamycin, rifaximin or vancomycin, which may be administered orally, intravenously, rectally or other method found useful by one of skill in the art, such as through a feeding tube or stoma. (R. K. Cleary [1998]; C. P. Kelly and J. T. LaMont, Clostridium difficile infection, Annu. Rev. Med. 49'375-90 [1998]; C. M. Reinke and C. R Messick, Update on Clostridium diffcile-induced colitis, Part 2, Am. J. Hosp. Pharm. 51(15):1892-1901 [1994]).

In certain embodiments, it is advantageous to administer an anti-inflammatory composition. Suitable anti-inflammatory drugs include, for example, steroidal anti-inflammatory agents, nonsteroidal anti-inflammatory agents, or combinations thereof. In some embodiments, anti-inflammatory drugs include, for example, alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, Clobetasol propionate, clobetasone butyrate, clopirac, cloticasone propionate, cormethasone acetate, cortodoxone, deflazacort, desonide, desoximetasone, dexamethasone dipropionate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, fuiraprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lomoxicam, loteprednol etabonate, meclofenamate sodium, meclofenamic acid, meclorisone dibutyrate, mefenamic acid, mesalamine, meseclazone, methylprednisolone suleptanate, momiflumate, nabumetone, naproxen, naproxen sodium, naproxol, nimazone, olsalazine sodium, orgotein, orpanoxin, oxaprozin, oxyphenbutazone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, sudoxicam, sulindac, suprofen, talmetacin, talniflumate, talosalate, tebufelone, tenidap, tenidap sodium, tenoxicam, tesicam, tesimide, tetrydamine, tiopinac, tixocortol pivalate, tolmetin, tolmetin sodium, triclonide, triflumidate, zidometacin, zomepirac sodium, aspirin (acetylsalicylic acid), salicylic acid, corticosteroids, glucocorticoids, tacrolimus, pimecorlimus, prodrugs thereof, co-drugs thereof, and combinations thereof.

### Durability of Response

Certain embodiments relate to the discovery that the dosing regime described herein of rifaximin results in a durability of response and amelioration of BD symptoms in subjects in need thereof. One embodiment is a use for treating bowel disease (BD) with a durability of antibiotic response, by administering a therapeutically effective amount of a rifamycin class antibiotic to a subject in need thereof, selecting subjects who respond to treatment after being treated for between about 1 and about 24 weeks, and removing a responding subject from treatment wherein after removal of treatment there is a durability of response. The selecting may be by a healthcare professional, by self selection or by selection of one in a position to decide or discern symptoms or to diagnose a response to the antibiotic. Removal of treatment comprises, for example, ceasing to administer, ceasing to recommend administration of the antibiotic, and/or advising responding subjects to stop taking the antibiotic.

Also described herein are methods for maintenance of remission of bowel disease in a subject comprising administering a therapeutically effective amount of rifaximin for at least 25 weeks to a subject in need thereof.

Yet another aspect relates to the treatment of a subject (e.g., mammal, human, horse, dog, cat) with rifaximin who is in need thereof. Identifying a subject in need of such treatment can be in the judgment of a subject or a health care professional and can be subjective (e.g. opinion) or objective (e.g. measurable by a test or diagnostic method).

Rifaximin may be used in various treatment regimes. These regimes may vary depending upon the subject and the type of treatment.

Rifaximin may be administered, for example, once a day, twice a day, three times a day, or four times a day. Rifaximin may be administered in doses, for example of from about between 50 mg BID to about 2500 mg TID. Another example is administering rifaximin from between about 600mg/day to about 3000mg/day. The rifaximin may be administered, for example, in tablet form, powered form, liquid for or in capsules.

Subjects in need thereof include subjects that are susceptible to BD, are in remission from BD, males and/or older subjects with long duration of disease, as disclosed further below.

As used herein, a therapeutically effective amount means an amount effective, when administered to a human or non-human subject, to provide a therapeutic benefit such as an amelioration of symptoms, e.g., an amount effective to decrease the symptoms of BDs, or maintenance of remission of a BD.

In certain embodiments, the rifaximin is administered to a subject from between about 1 week to about 6 weeks in duration, from between about 8 weeks to about 12 weeks in duration, or from between 1 day to about 7 days. The rifaximin may be administered intermittently or continuously during the course of treatment. Length of treatment may vary depending on the type and length of disease and the proper length of treatment may be easily determined by one of skill in the art having the benefit of this disclosure.

For any of the embodiments, rifaximin may be administered, for example, once daily, twice daily, three times daily, or four times daily to a subject. In some particularly preferred methods of the present invention comprise administering the rifaximin twice daily to the subject because it may, for example, minimize the side effects and increase patient compliance.

Dosages, according to certain preferred embodiments, range from between about 50 to about 6000 mg of rifaximin administered daily. For example, a dose of 3000 mg may be administered to a subject twice daily. Other appropriate dosages for methods according to this invention may be determined by health care professionals or by the subject. The amount of rifaximin administered daily may be increased or decreased based on the weight, age, health, sex or medical condition of the subject. One of skill in the art would be able to determine the proper dose for a subject based on this disclosure.

Indications include a subject receiving radiotherapy, chemotherapy, and/or surgical procedure as a result of treatment for cancer of the cervix, prostate, appendix, colon, intestine, rectum, or other gastrointestinal malignancy, or prostatectomy.

According to certain embodiments, rifaximin may be administered in combination with other compounds, including for example, chemotherapeutic agents, anti-inflammatory agents, anti-pyretic agents radiosensitizing agents, radioprotective agents, urologic agents, anti-emetic agents, and/or anti-diarrheal agents. for example, cisplatin, carboplatin, docetaxel, paclitaxel, flurouracil, capecitabine, gemcitabine, irinotecan, topotecan, etoposide, mitomycin, gefitinib, vincristine, vinblastine, doxorubicin, cyclophosphamide, celecoxib, rofecoxib, valdecoxib, ibuprofen, naproxen, ketoprofen, dexamethasone, prednisone, prednisolone, hydrocortisone, acetaminophen, misonidazole, amifostine, tamsulosin, phenazopyridine, ondansetron, granisetron, alosetron, palonosetron, promethazine, prochlorperazine, trimethobenzamide, aprepitant, diphenoxylate with atropine, and/or loperamide.

The uses disclosed herein are also useful for protecting a subject against radiation induced enteritis by administering to a subject in need thereof a therapeutically effective amount of rifaximin. For example, prophylactic doses may be administered prior to a patient undergoing radiation.

The uses disclosed herein are useful for protecting a subject against radiation induced injury to the mucosa of the colon, as well as against radiation induced colorectal inflammation by administering to a subject in need thereof a therapeutically effective amount of rifaximin.

### Pharmaceutical Preparations

The disclosure also provides pharmaceutical compositions, comprising an effective amount of a GI cleanser and rifaximin. Rifaximin, which may be found, for example, as a polymorph, salt, hydrate or as an amorphous form along with may be formulated with a pharmaceutically acceptable carrier. In a further embodiment, the effective amount is effective to treat a bacterial infection, e.g., small intestinal bacterial overgrowth, Crohn's disease, hepatic encephalopathy, antibiotic associated colitis, and/or diverticular disease.

For examples of the use of rifaximin to treat Travelers' diarrhea, see Infante RM, Ericsson CD, Zhi-Dong J, Ke S, Steffen R, Riopel L, Sack DA, DuPont, HL. Enteroaggregative Escherichia coli Diarrhea in Travelers: Response to Rifaximin Therapy. Clinical Gastroenterology and Hepatology. 2004;2:135-138; and Steffen R, M.D., Sack DA, M.D., Riopel L, Ph.D., Zhi-Dong J, Ph.D., Sturchler M, M.D., Ericsson CD, M.D., Lowe B, M.Phil., Waiyaki P, Ph.D., White M, Ph.D., DuPont HL, M.D. Therapy of Travelers' Diarrhea With Rifaximin on Various Continents. The American Journal of Gastroenterology. May 2003, Volume 98, Number 5.

One aspect of the disclosure provides pharmaceutical compositions comprising a GI cleanser and an antibiotic in a pharmaceutically acceptable carrier. The GI cleanser may be selected, for example on the basis of the subject's tolerability of sodium phosphate, taste preference or method of administering (liquid v/s solid), desired amounts of systemic adsorption, dissolution profile, desired location in the digestive tract to be treated, and the like. The pharmaceutical composition further comprises excipients, for example, one or more of a diluting agent, binding agent, lubricating agent, disintegrating agent, coloring agent, flavoring agent or sweetening agent. Antibiotic compositions may be formulated for selected coated and uncoated tablets, hard and soft gelatin capsules, sugar-coated pills, lozenges, wafer sheets, pellets and powders in sealed packet. For example, compositions may be formulated for topical use, for example, ointments, pomades, creams, gels and lotions.

In an aspect, the antibiotic is administered to the subject using a pharmaceutically-acceptable formulation, e.g., a pharmaceutically-acceptable formulation that provides sustained delivery of the antibiotic to a subject for at least 4, hours, 6 hours, 8 hours, 12 hours, 18 hours, 24 hours, 36 hours, 48 hours, one week, two weeks, three weeks, or four weeks after the pharmaceutically-acceptable formulation is administered to the subject.

In certain aspects, these pharmaceutical compositions are suitable for topical or oral administration to a subject. In other aspects, as described in detail below, the pharmaceutical compositions for use in the present invention may be specially formulated for administration in solid or liquid form, including those adapted for the following: (1) oral administration, for example, drenches (aqueous or non-aqueous solutions or suspensions), tablets, boluses, powders, granules, pastes; (2) parenteral administration, for example, by subcutaneous, intramuscular or intravenous injection as, for example, a sterile solution or suspension; (3) topical application, for example, as a cream, ointment or spray applied to the skin; (4) intravaginally or intrarectally, for example, as a pessary, cream or foam; or (5) aerosol, for example, as an aqueous aerosol, liposomal preparation or solid particles containing the compound.

The phrase "pharmaceutically acceptable" refers to those antibiotics and GI cleansers described herein, compositions containing such compounds, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" includes pharmaceutically-acceptable material, composition or vehicle, such as a liquid or solid filler, diluent, excipient, solvent or encapsulating material, involved in carrying or transporting the subject chemical from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Compositions containing an antibiotic include those suitable for oral, nasal, topical (including buccal and sublingual), rectal, vaginal, aerosol and/or parenteral administration. The compositions may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred percent, this amount will range from about 1% to about 99 % of active ingredient, preferably from about 5 % to about 70 %, most preferably from about 10 % to about 30 %.

Methods of preparing these compositions include the step of bringing into association an antibiotic with the carrier and, optionally, one or more accessory ingredients. In general, the formulations are prepared by uniformly and intimately bringing into association an antibiotic with liquid carriers, or finely divided solid carriers, or both, and then, if necessary, shaping the product.

Compositions suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of an antibiotic(s) as an active ingredient. A compound may also be administered as a bolus, electuary or paste.

The amorphous form, Form α, Form β, Form γ, Form δ, Form ε, Form ζ, or Form η polymorph of rifaximin can be advantageously used in the production of medicinal preparations having antibiotic activity, containing rifaximin, for both oral and topical use. The medicinal preparations for oral use will contain one or more of an amorphous form, Form α, Form β, Form γ, Form δ, Form ε, Form ξ, or Form η polymorph of rifaximin together with the usual excipients, for example diluting agents such as mannitol, lactose and sorbitol; binding agents such as starches, gelatines, sugars, cellulose derivatives, natural gums and polyvinylpyrrolidone; lubricating agents such as talc, stearates, hydrogenated vegetable oils, polyethylenglycol and colloidal silicon dioxide; disintegrating agents such as starches, celluloses, alginates, gums and reticulated polymers; colouring, flavouring and sweetening agents.

Aspects of the disclosure relate to all of the solid preparations administrable by the oral route, for instance coated and uncoated tablets, of soft and hard gelatin capsules, sugar-coated pills, lozenges, wafer sheets, pellets and powders in sealed packets or other containers.

The medicinal preparations for topical use can contain one or more of an amorphous form, Form α, Form β, Form γ, Form δ, Form ε, Form ζ, or Form η polymorph of rifaximin together with usual excipients, such as white petrolatum, white wax, lanoline and derivatives thereof, stearylic alcohol, propylene glycol, sodium lauryl sulfate, ethers of fatty polyoxyethylene alcohols, esters of fatty polyoxyethylene acids, sorbitan monostearate, glyceryl monostearate, propylene glycol monostearate, polyethylene glycols, methylcellulose, hydroxymethyl propylcellulose, sodium carboxymethylcellulose, colloidal aluminium and magnesium silicate, sodium alginate.

One embodiment relates to all of the topical preparations, for instance ointments, pomades, creams, gels and lotions.

In solid dosage forms for oral administration (capsules, tablets, pills, dragees, powders, granules and the like), the active ingredient is typically mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such as talc, calcium stearate, magnesium stearate, solid polyethylene glycols, sodium lauryl sulfate, and mixtures thereof; and (10) colouring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered active ingredient moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral or rectal administration of the antibiotic(s) include pharmaceutically-acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

In addition to inert diluents, the oral compositions can include adjuvants such as wetting agents, emulsifying and suspending agents, sweetening, flavoring, coloring, perfuming and preservative agents.

Suspensions, in addition to the active antibioticsagent (e.g., the GI cleanser and/or the antibiotic) may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Pharmaceutical compositions for rectal administration may be presented as a suppository, which may be prepared by mixing one or more antibiotics with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active agent.

Compositions which are suitable for vaginal administration can include pessaries, tampons, creams, gels, pastes, foams or spray formulations containing such carriers as are known in the art to be appropriate.

Dosage forms for the topical or transdermal administration of an antibiotic(s) can include powders, sprays, ointments, pastes, creams, lotions, gels, solutions, patches and inhalants. The active antibiotics may be mixed under sterile conditions with a pharmaceutically-acceptable carrier, and with any preservatives, buffers, or propellants which may be required.

The ointments, pastes, creams and gels may contain, in addition to antibiotics, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a antibiotics, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

The antibiotic(s) can be alternatively administered by aerosol. This is accomplished by preparing an aqueous aerosol, liposomal preparation or solid particles containing the compound. A non-aqueous (e.g., fluorocarbon propellant) suspension could be used. Sonic nebulizers are preferred because they minimize exposing the agent to shear, which can result in degradation of the compound.

Ordinarily, an aqueous aerosol is made by formulating an aqueous solution or suspension of the agent together with conventional pharmaceutically-acceptable carriers and stabilizers. The carriers and stabilizers vary with the requirements of the particular compound, but typically include non-ionic surfactants (Tweens, Pluronics, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

Transdermal patches have the added advantage of providing controlled delivery of an antibiotic(s) to the body. Such dosage forms can be made by dissolving or dispersing the agent in the proper medium. Absorption enhancers can also be used to increase the flux of the active ingredient across the skin. The rate of such flux can be controlled by either providing a rate controlling membrane or dispersing the active ingredient in a polymer matrix or gel.

Pharmaceutical compositions suitable for parenteral administration can comprise one or more antibiotics in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and non-aqueous carriers which may be employed in the pharmaceutical compositions can include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of antibiotics in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

When the antibiotic(s) are administered as pharmaceuticals, to humans and animals, they can be given *per se* or as a pharmaceutical composition containing, for example, 0.1 to 99.5% (more preferably, 0.5 to 90%) of active ingredient in combination with a pharmaceutically-acceptable carrier.

Regardless of the route of administration selected, the antibiotic(s), which may be used in a suitable hydrated form, and/or the pharmaceutical compositions of the present invention, are formulated into pharmaceutically-acceptable dosage forms by conventional methods known to those of skill in the art.

Actual dosage levels and time course of administration of the active ingredients in the pharmaceutical compositions may be varied so as to obtain an amount of the active ingredient which is effective to achieve the desired therapeutic response for a particular patient, composition, and mode of administration, without being toxic to the patient. An exemplary dose range is from 100 to 3000 mg per day.

A preferred dose of the antibiotic for the present invention is the maximum that a patient can tolerate without developing serious side effects. Preferably, the antibiotic of the present invention is administered at a concentration of about 1 mg to about 200 mg per kilogram of body weight, about 10 - about 100 mg/kg or about 40 mg - about 80 mg/kg of body weight. Ranges intermediate to the above-recited values are also intended to be part .

In combination therapy treatment, both the compounds and the other drug agent(s) are administered to subjects (e.g., humans, male or female) by appropriate means. The agents may be administered in a single dosage form or in separate dosage forms. Effective amounts of the other therapeutic agents for particular purposes are well known to those skilled in the art. However, it is well within the skilled artisan's purview to determine the other therapeutic agent's optimal effective-amount range. In one aspect in which another therapeutic agent is administered to a subject, the effective amount of the compound is less than its effective amount in case the other therapeutic agent is not administered. In another aspect, the effective amount of the agent is less than its effective amount in case the compound is not administered. In this way, undesired side effects associated with high doses of either agent may be minimized. Other potential advantages (including without limitation improved dosing regimens and/or reduced drug cost) will be apparent to those skilled in the art.

In various aspects, the therapies (e.g., prophylactic or therapeutic agents) are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In preferred embodiments, two or more therapies are administered within the same patient's visit.

In certain embodiments, one or more of the antibiotics and one or more other therapies (e.g., prophylactic or therapeutic agents) are cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time, optionally, followed by the administration of a third therapy (e.g., prophylactic or therapeutic agent) for a period of time and so forth, and repeating this sequential administration, e.g., the cycle in order to reduce the development of resistance to one of the therapies, to avoid or reduce the side effects of one of the therapies, and/or to improve the efficacy of the therapies.

In certain embodiments, the administration of the same compounds may be repeated and the administrations may be separated by at least 1 day, 2 days, 3 days, 5 days, 10 days, 15 days, 30 days, 45 days, 2 months, 75 days, 3 months, or at least 6 months. In other embodiments, the administration of the same therapy (e.g., prophylactic or therapeutic agent) other than an antibiotic may be repeated and the administration may be separated by at least at least 1 day, 2 days, 3 days, 5 days, 10 days, 15 days, 30 days, 45 days, 2 months, 75 days, 3 months, or at least 6 months.

Certain indications may require longer treatment times. For example, travelers' diarrhea treatment may only last from between about 12 hours to about 72 hours, while a treatment for Crohn's disease may be from between about 1 day to about 3 months.

Bowel related disorders include one or more of irritable bowel syndrome, travelers' diarrhea, small intestinal bacterial overgrowth, Crohn's disease, enteritis or colitis.

The length of treatment for a particular bowel disorder will depend in part on the disorder. For example, travelers' diarrhea may only require treatment duration of 12 to about 72 hours, while Crohn's disease may require treatment durations from about 2 days to 3 months. Dosages of rifaximin will also vary depending on the diseases state. Proper dosage ranges are provided herein infra.

The identification of those subjects who are in need of prophylactic treatment for bowel disorder is well within the ability and knowledge of one skilled in the art. Certain of the ways for identification of subjects which are at risk of developing a bowel disorder which can be treated by the subject treatment are appreciated in the medical arts, such as family history, travel history and expected travel plans, the presence of risk factors associated with the development of that disease state in the subject. A clinician skilled in the art can readily identify such candidate subjects, by the use of, for example, clinical tests, physical examination and medical/family/travel history.

An antibiotic can be administered at the initial dosage of from about 0.001 mg/kg to about 1000 mg/kg daily. A daily dose range of from about 0.01 mg/kg to about 500 mg/kg, from about 0.1 mg/kg to about 200 mg/kg, from about 1 mg/kg to about 100 mg/kg, or from about 10 mg/kg to about 50 mg/kg, can be used. The dosages, however, may be varied depending upon the requirements of the individual, the severity of the BD symptoms, and the antibiotic being employed. For example, dosages can be empirically determined considering the severity of IBS symptoms in an individual classified as having IBS according to the methods described herein. The dose administered to an individual, in the context of the present invention, should be sufficient to affect a beneficial therapeutic response in the individual over time. The size of the dose can also be determined by the existence, nature, and extent of any adverse side-effects that accompany the administration of a particular antibiotic in an individual. Determination of the proper dosage for a particular situation is within the skill of the practitioner. Generally, treatment is initiated with smaller dosages which are less than the optimum dose of the antibiotic. Thereafter, the dosage is increased by small increments until the optimum effect under circumstances is reached. For convenience, the total daily dosage may be divided and administered in portions during the day, if desired.

In certain embodiments, other therapeuctic compositions, such as "IBS drugs" may be co-administered prior to, during, between or after the administration of the GI cleanser and the antibiotic. As used herein, the tenm "IBS drug" includes, for example, all pharmaceutically acceptable forms of a drug that is useful for treating one or more symptoms associated with IBS. For example, the IBS drug can be in a racemic or isomeric mixture, a solid complex bound to an ion exchange resin, or the like. In addition, the IBS drug can be in a solvated form. The term "IBS drug" is also intended to include all pharmaceutically acceptable salts, derivatives, and analogs of the IBS drug being described, as well as combinations thereof. For example, the pharmaceutically acceptable salts of an IBS drug include the tartrate, succinate, tartarate, bitartarate, dihydrochloride, salicylate, hemisuccinate, citrate, maleate, hydrochloride, carbamate, sulfate, nitrate, and benzoate salt forms thereof, as well as combinations thereof and the like. Any form of an IBS drug is suitable for use in the present invention, e.g., a pharmaceutically acceptable salt of an IBS drug, a free base of an IBS drug, or a mixture thereof.

Suitable drugs that are useful for treating one or more symptoms associated with IBS include serotonergic agents, antidepressants, chloride channel activators, chloride channel blockers, guanylate cyclase agonists, antibiotics, opioids, neurokinin antagonists, antispasmodic or anticholinergic agents, belladonna alkaloids, barbiturates, glucagon-like peptide-1 (GLP-1) analogs, corticotropin releasing factor (CRF) antagonists, probiotics, free bases thereof, pharmaceutically acceptable salts thereof, derivatives thereof, analogs thereof, and combinations thereof. Other IBS drugs include bulking agents, dopamine antagonists, carminatives, tranquilizers, dextofisopam, phenytoin, timolol, and diltiazem. Serotonergic agents are useful for the treatment of IBS symptoms such as constipation, diarrhea, and/or alternating constipation and diarrhea. Examples of serotonergic agents are described in Cash et al., Aliment. Pharmacol. Ther., 22:1047-1060 (2005), and include 5-HT₃ receptor agonists (e.g., MKC-733, etc.), 5-HT₄ receptor agonists (e.g., tegaserod (Zelnorm TM), prucalopride, AG1-001, etc.), 5-HT₃ receptor antagonists (e.g., alosetron (Lotronex.RTM.), cilansetron, ondansetron, granisetron, dolasetron, ramosetron, palonosetron, E-3620, DDP-225, DDP-733, etc.), mixed 5-HT₃ receptor antagonists/5-HT₄ receptor agonists (e.g., cisapride, mosapride, renzapride, etc.), free bases thereof, pharmaceutically acceptable salts thereof, derivatives thereof, analogs thereof, and combinations thereof. Additionally, amino acids like glutamine and glutamic acid which regulate intestinal permeability by affecting neuronal or glial cell signaling can be administered to treat patients with IBS. Antidepressants such as selective serotonin reuptake inhibitor (SSRI) or tricyclic antidepressants are particularly useful for the treatment of IBS symptoms such as abdominal pain, constipation, and/or diarrhea. Non-limiting examples of SSRI antidepressants include citalopram, fluvoxamine, paroxetine, fluoxetine, sertraline, free bases thereof, pharmaceutically acceptable salts thereof, derivatives thereof, analogs thereof, and combinations thereof. Examples of tricyclic antidepressants include desipramine, nortriptyline, protriptyline, amitriptyline, clomipramine, doxepin, imipramine, trimipramine, maprotiline, amoxapine, clomipramine, free bases thereof, pharmaceutically acceptable salts thereof, derivatives thereof, analogs thereof, and combinations thereof. Chloride channel activators are useful for the treatment of IBS symptoms such as constipation. An example of a chloride channel activator is lubiprostone (Amitiza TM), a free base thereof, a pharmaceutically acceptable salt thereof, a derivative thereof, or an analog thereof. In addition, chloride channel blockers such as crofelemer are useful for the treatment of IBS symptoms such as diarrhea. Guanylate cyclase agonists such as MD-1100 are useful for the treatment of constipation associated with IBS (see, e.g., Bryant et al., Gastroenterol., 128:A-257 (2005)). Antibiotics such as neomycin can also be suitable for use in treating constipation associated with IBS (see, e.g., Park et al., Gastroenterol., 128:A-258 (2005)). Non-absorbable antibiotics like rifaximin (Xifaxan.TM.) are suitable to treat small bowel bacterial overgrowth and/or constipation associated with IBS (see, e.g., Sharara et al., Am. J. Gastroenterol., 101:326-333 (2006)).

Opioids such as kappa opiods (e.g., asimadoline) may be useful for treating pain and/or constipation associated with IBS. Neurokinin (NK) antagonists such as talnetant, saredutant, and other NK2 and/or NK3 antagonists may be useful for treating IBS symptoms such as oversensitivity of the muscles in the colon, constipation, and/or diarrhea. Antispasmodic or anticholinergic agents such as dicyclomine may be useful for treating IBS symptoms such as spasms in the muscles of the gut and bladder. Other antispasmodic or anticholinergic agents such as belladonna alkaloids (e.g., atropine, scopolamine, hyoscyamine, etc.) can be used in combination with barbiturates such as phenobarbital to reduce bowel spasms associated with IBS. GLP-1 analogs such as GTP-010 may be useful for treating IBS symptoms such as constipation. CRF antagonists such as astressin and probiotics such as VSL#3 TM may be useful for treating one or more IBS symptoms. One skilled in the art will know of additional IBS drugs currently in use or in development that are suitable for treating one or more symptoms associated with IBS.

An individual can also be monitored at periodic time intervals to assess the efficacy of a certain therapeutic regimen once a sample from the individual has been classified as an IBS sample. For example, the levels of certain markers change based on the therapeutic effect of a treatment such as a drug. The patient is monitored to assess response and understand the effects of certain drugs or treatments in an individualized approach. Additionally, patients may not respond to a drug, but the markers may change, suggesting that these patients belong to a special population (not responsive) that can be identified by their marker levels. These patients can be discontinued on their current therapy and alternative treatments prescribed.

### Diagnosis

In certain aspects, the BD may be diagnosed. For example, as those described in US Patent Application No. 20080085524. Exemplary diagnostic tests for IBS, for example, include the hydrogen breath test or a blood test diagnostic. Diagnosis may also be based on a subject's symptoms.

### Article of Manufacture

Another aspect of the disclosure includes articles of manufacture that comprise, for example, a container holding a GI cleanser pharmaceutical composition and an antibiotic pharmaceutical composition suitable for oral or topical administration in combination with printed labeling instructions providing a discussion of when a particular composition and dosage form should be administered. Exemplary dosage forms and administration protocols are described infra. The composition will be contained in any suitable container capable of holding and dispensing the dosage form and which will not significantly interact with the composition and will further be in physical relation with the appropriate labeling. The labeling instructions will be consistent with the treatment as described hereinbefore. The labeling may be associated with the container by any means that maintain a physical proximity of the two, by way of non-limiting example, they may both be contained in a packaging material, such as a box or plastic shrink wrap or may be associated with the instructions being bonded to the container such as with glue that does not obscure the labeling instructions or other bonding or holding means.

Another aspect is an article of manufacture that comprises a container containing a pharmaceutical composition comprising a GI cleanser and rifaximin wherein the container holds a GI cleanser in ready to drink or administer formulation and a rifaximin composition in unit dosage form and is associated with printed labeling instructions advising the subject how to take the composition.

Packaged compositions are also provided, and may comprise a therapeutically effective amount of a GI cleanser and of rifaximin. Rifaximin and a pharmaceutically acceptable carrier or diluent, wherein the composition is formulated for treating a subject suffering from or susceptible to a bowel disorder, and packaged with instructions to treat a subject suffering from or susceptible to a bowel disorder.

The antibiotic used in the present invention is rifaximin. Rifaximin exists in several different, distinct forms. Such forms are, for example, described in US 7,045,620 B1; USSNs 11/135,651; 11/658,702; 11/873,841; and US 61/031,329; filed 25 Feb 2008, all of which are incorporated herein by reference in their entirety. US 61/031,329 describes Form ζ, Form η and additional amorphous forms. The polymorph Form ζ exhibits an X-ray powder diffraction pattern having characteristic peaks expressed in degrees 2θ (+/- 0.20 degree θ) at 4.7 (doublet), 7.6 (doublet), and 9.5 degrees 2-θ; or 4.7 (doublet), 7.3, and 8.2 degrees 2-θ; or 7.6 (doublet), 8.6, and 10.5 degrees 2-θ; or 8.2, 8.6, and 9.5 degrees 2-θ; or 10.2 (triplet), 12.6 (quintet), and 13.2 (doublet) degrees 2-θ; or 7.3, 10.5, and 12.9 (doublet) degrees 2-θ; or 7.3, 7.6 (doublet), 8.2, 8.6 degrees 2-θ; or 4.7 (doublet), 7.3, 7.6 (doublet), 9.5, and 10.5 degrees 2-θ; or 8.2, 8.6, 9.5, 10.2 (triplet), and 10.5 degrees 2-θ; or 8.6, 9.5, 10.2 (triplet), 10.5, and 11.2 (doublet) degrees 2-θ; or 4.7 (doublet), 6.3, 6.4, 7.3, 7.6 (doublet), 8.2, 8.6, 9.5, 10.2 (triplet), 10.5, 11.2 (doublet), 11.9 (doublet), 12.2 (weak), 12.6 (quintet), 12.9 (doublet), 13.2 (doublet) degrees 2-θ.

According to one aspect, Form η exhibits an X-ray powder diffraction pattern having characteristic peaks expressed in degrees 2θ (+/- 0.20 degree θ) at 6.1, 7.3, and 7.5 degrees 2-θ; or 6.1, 7.3, and 7.9 degrees 2-θ; or 6.1, 7.3, and 8.8 degrees 2-θ; or 6.1, 7.3, and 12.7 degrees 2-θ; or 6.1, 7.5, and 8.8 degrees 2-θ; or 6.1, 7.5, and 7.9 degrees 2-0; or 5.3, 6.1, and 7.3 degrees 2-θ; or 5.3, 6.1, and 7.9 degrees 2-θ; or 5.3, 6.1, and 12.7 degrees 2-θ; or 5.3, 6.1, and 7.5 degrees 2-θ; or 5.3, 6.1, and 8.8 degrees 2-θ; or 6.1, 7.3, 7.5, 7.9, 8.8, and 12.7 degrees 2-θ; or 5.3, 6.1, 7.3, 7.5, 7.9, 8.8, 12.7 degrees 2-θ; or 5.3, 6.1, 7.3, 7.9, 8.8, and 12.7 degrees 2-θ; or 5.3, 6.1, 7.3, 7.5, 8.8, and 12.7 degrees 2-θ; or 5.3, 6.1, 7.3, 7.5, 7.9, 8.8, and 12.7 degrees 2-θ.

According to one aspect, a polymorph amorphous form exhibits an X-ray powder diffraction pattern having characteristic peaks expressed in degrees 2θ (+/- 0.20 degree θ) at 7.3 (approximate halo maximum), 11.3-17.8 (amorphous halo range), and 15.8 (approximate halo maximum) degrees 2-θ; or 5.1-10.1 (amorphous halo range), 11.3-17.8 (amorphous halo range), and 15.8 (approximate halo maximum) degrees 2-θ; or 5.1-10.1 (amorphous halo range), 7.3 (approximate halo maximum), and 11.3-17.8 (amorphous halo range) degrees 2-θ; or 5.1-10.1 (amorphous halo range), 7.3 (approximate halo maximum), and 15.8 (approximate halo maximum) degrees 2-θ; or 5.1-10.1 (amorphous halo range), 7.3 (approximate halo maximum), 11.3-17.8 (amorphous halo range), 15.8 (approximate halo maximum) degrees 2-θ. Forms ζ, η, and amorphous are further described in USSN 61/031,329.

### EXAMPLES

This example relates to a study of three rifaximin doses in subjects with dIBS. Subjects were randomized to receive daily BID doses of rifaximin 275 mg, 550 mg, or 1100 mg for 14 days. A fifth group of subjects received rifaximin 550 mg BID for a period of 28 days. Subjects were questioned on the relief of overall IBS symptoms and bloating. Adequate relief of IBS related symptoms (SGA) and IBS-related bloating (IBS-B) were tested, a dose of 550 mg BID for 2 weeks demonstrated statistically significant relief in each of the endpoints when compared to placebo treated subjects. The analyses defined success as a "yes" response to questions regarding adequate relief of SGA or IBS-B in at least 2 out of the final 3 weeks of the double-blind treatment period.

Predictors of response analyses showed that the response was similar across some subgroups however, there were qualitative differences. Supplementary analyses on predictors of response demonstrated that age (older subjects and those with a longer IBS duration); sex (males) and baseline severity (mild to moderate symptoms) were predictors of response. Baseline severity was determined using 7-point Lickert scales during screening for Abdominal Pain/Discomfort and Bloating, and the number, type (normal, hard, loose) and urgency of bowel movements.

Duration of effect was assessed in a 12 week follow-up period. Subjects that responded in the 4 week double-blind treatment period were followed for an additional 3 months. Fifty-three (53) subjects from the 550 mg 2w and 59 subjects from the placebo-treated groups participated in this study phase. The subjects in the placebo group had a greater rate of decline in response than the 550 mg BID 2w group, demonstrating that subjects treated with rifaximin (RFX) had a better chance of maintaining symptom relief than their placebo treated counterparts.

### Daily Symptom Score

Subjects recorded the following information on dIBS symptoms daily throughout the duration of the study:
- Number of normal stools/day;
- Number of hard and lumpy stools/day;
- Number of loose or watery stools/day;
- Number of loose or watery stools/day with the symptom of urgency;
- How bothersome is abdominal pain and discomfort? [7-point response scale: 0 (not at all) to 6 (a very great decal)];
- How bothersome is bloating? [(7-point response scale: 0 (not at all) to 6 (a very great decal)].

Weekly summary variables were computed by averaging the recorded values over all pretreatment days or over the seven days preceding the time point of interest for post-treatment assessments. For example, baseline included Days -10 to 0, Week 1 included Days 1 to 7, Week 2 included Days 8 to 14, and so forth through Week 16. Changes from baseline variables were computed for each weekly summary score.

As shown in Table 1, subjects having a purgative prior to treatment with rifaximin as described below in the Examples, were much more likely to have a treatment effect than those who did not have a cleanser prior to beginning treatment.

**Table 1:**

| | **Thresholds** | **Treatment effect (IBS Sx, Bloating)** |
|---|---|---|
| GI Cleanser Prior to Rifaximin Administration | Y vs. | 11.6%, 13.8% |
| | N | 4.7%, -0.9% |

**Table 2:**

| Efficacy Analysis of Adequate Relief of IBS Symptoms and Bloating at the End of the Treatment Phase | | | |
|---|---|---|---|
| | | PBO 4w | RFX 550 2w |
| Had a Screening Colonoscopy? | | (N=197) | (N=191) |
| | | n (%) | n (%) |
| Yes | Adequate Relief of IBS Symptoms [1] | | |
| | Success | 42 (44.7%) | 54 (56.3%) |
| | Failure | 52 (55.3%) | 42 (43.8%) |
| | Adequate Relief of Bloating [2] | | |
| | Success | 38 (40.4%) | 52 (54.2%) |
| | Failure | 56 (59.6%) | 44 (45.8%) |
| No | Adequate Relief of IBS Symptoms [1] | | |
| | Success | 45 (43.7%) | 46 (48.4%) |
| | Failure | 58 (56.3%) | 49 (51.6%) |
| | Adequate Relief of Bloating [2] | | |
| | Success | 40 (38.8%) | 36 (37.9%) |
| | Failure | 63 (61.2%) | 59 (62.1%) |

| | | | |
|---|---|---|---|
| [1] Subjects achieved success if they reported a 'yes' response to whichever question about IBS symptoms was posed by the IVR system(e.g. adequate relief or control) for =2 out of the 3 final treatment weeks. [2] Subjects achieved success if they reported a 'yes' response to whichever question about symptoms of bloating was posed by the IVR system (e.g. adequate relief or control) for =2 out of the 3 final treatment weeks. | | | |

The tables below, Tables 3 - 8, demonstrate that subjects who had a screening colonoscopy, and thus given a GI cleanser prior to being treated with rifaximin, had a higher rate of success in relief of symptoms. This shows that the administration of a GI cleanser prior to administration of an antibiotic is efficacious to treat a BD.

**Table 3:**

| Efficacy Analysis of Adequate Relief of IBS Symptoms and Bloating at the End of the Treatment Phase | | | | | | |
|---|---|---|---|---|---|---|
| | | PBO 4w | RFX 275 2w | RFX 550 2w | RFX 1100 2w | RFX 550 4w |
| | | (N=197) | (N=95) | (N=191) | (N=99) | (N=98) |
| | | n (%) | n (%) | n (%) | n (%) | n (%) |
| Had a Screening Colonoscopy? | | | | | | |
| Yes | Adequate Relief of IBS Symptoms [1] | | | | | |
| | Success | 42 (44.7%) | 19 (45.2%) | 54 (56.3%) | 23 (42.6%) | 19 (46.3%) |
| | Failure | 52 (55.3%) | 23 (54.8%) | 42 (43.8%) | 31 (57.4%) | 22 (53.7%) |
| | Adequate Relief of Bloating [2] | | | | | |
| | Success | 38 (40.4%) | 18 (42.9%) | 52 (54.2%) | 23 (42.6%) | 18 (43.9%) |
| | Failure | 56 (59.6%) | 24 (57.1%) | 44 (45.8%) | 31 (57.4%) | 23 (56.1%) |
| No | Adequate Relief of IBS Symptoms [1] | | | | | |
| | Success | 45 (43.7%) | 21 (39.6%) | 46 (48.4%) | 18 (40.0%) | 22 (38.6%) |
| | Failure | 58 (56.3%) | 32 (60.4%) | 49 (51.6%) | 27 (60.0%) | 35 (61.4%) |
| | Adequate Relief of Bloating [2] | | | | | |
| | Success | 40 (38.8%) | 17 (32.1%) | 36 (37.9%) | 15 (33.3%) | 20 (35.1%) |
| | Failure | 63 (61.2%) | 36 (67.9%) | 59 (62.1%) | 30 (66.7%) | 37 (64.9%) |

| | | | | | | |
|---|---|---|---|---|---|---|
| [1] Subjects achieved success if they reported a 'yes_{'} response to whichever question about IBS symptoms was posed by the IVR system(e.g. adequate relief or control) for =2 out of the 3 final treatment weeks. [2] Subjects achieved success if they reported a 'yes' response to whichever question about symptoms of bloating was posed by the IVR system (e.g. adequate relief or control) for =2 out of the 3 final treatment weeks. | | | | | | |

**Table 4:**

| Efficacy Analysis of Adequate Relief of IBS Symptoms and Bloating at the End of the Treatment Phase | | | | | | |
|---|---|---|---|---|---|---|
| | | PBO 4w | RFX 275 2w | RFX 550 2w | RFX 1100 2w | RFX 550 4w |
| | | (N=197) | (N=95) | (N=191) | (N=99) | (N=98) |
| | | n (%) | n (%) | n (%) | n (%) | n (%) |
| Screening Colonoscopy | | | | | | |
| <=26 Days | Adequate Relief of IBS Symptoms [1] | | | | | |
| | Success | 23 (45.1%) | 7 (38.9%) | 24 (54.5%) | 17 (47.2%) | 14 (56.0%) |
| | Failure | 28 (54.9%) | 11 (61.1%) | 20 (45.5%) | 19 (52.8%) | 11 (44.0%) |
| | Adequate Relief of Bloating [2] | | | | | |
| | Success | 23 (45.1%) | 8 (44.4%) | 26 (59.1%) | 16 (44.4%) | 13 (52.0%) |
| | Failure | 28 (54.9%) | 10 (55.6%) | 18 (40.9%) | 20 (55.6%) | 12 (48.0%) |
| >26 Days | Adequate Relief of IBS Symptoms [1] | | | | | |
| | Success | 19 (44.2%) | 12 (50.0%) | 30 (57.7%) | 6 (33.3%) | 5 (31.3%) |
| | Failure | 24 (55.8%) | 12 (50.0%) | 22 (42.3%) | 12 (66.7%) | 11 (68.8%) |
| | Adequate Relief of Bloating [2] | | | | | |
| | Success | 15 (34.9%) | 10 (41.7%) | 26 (50.0%) | 7 (38.9%) | 5 (31.3%) |
| | Failure | 28 (65.1%) | 14 (58.3%) | 26 (50.0%) | 11 (61.1%) | 11 (68.8%) |

| | | | | | | |
|---|---|---|---|---|---|---|
| [1] Subjects achieved success if they reported a 'yes' response to whichever question about IBS symptoms was posed by the IVR system(e.g. adequate relief or control) for =2 out of the 3 final treatment weeks. [2] Subjects achieved success if they reported a 'yes' response to whichever question about symptoms of bloating was posed by the IVR system (e.g. adequate relief or control) for =2 out of the 3 final treatment weeks. | | | | | | |

**Table 5:**

| Screening Colonoscopy Baseline Characteristics | | | | | |
|---|---|---|---|---|---|
| | PBO 4w | RFX 275 2w | RFX 550 2w | RFX 1100 2w | RFX 550 4w |
| | (N=94) | (N=42) | (N= 96) | (N=54) | (N=41) |
| | n (%) | n (%) | n (%) | n (%) | n (%) |
| Days of colonoscopy before Treatment | | | | | |
| n | 94 | 42 | 96 | 54 | 41 |
| Mean | 25.9 | 30.6 | 28.2 | 27.0 | 26.2 |
| SD | 7.81 | 11.18 | 8.90 | 20.02 | 8.30 |
| Median | 26.0 | 29.0 | 27.0 | 25.0 | 25.0 |
| Min | 6 | 15 | 8 | -23 | 8 |
| Max | 49 | 82 | 63 | 145 | 46 |
| Age | | | | | |
| n | 94 | 42 | 96 | 54 | 41 |
| Mean | 46.1 | 46.3 | 40.6 | 41.1 | 43.7 |
| SD | 13.77 | 14.24 | 11.70 | 13.82 | 13.92 |
| Median | 48.5 | 44.5 | 40.0 | 39.5 | 45.0 |
| Min | 20 | 23 | 19 | 22 | 19 |
| Max | 81 | 78 | 72 | 73 | 78 |
| Sex | | | | | |
| Male | 23 (24.5%) | 5 (11.9%) | 31 (32.3%) | 17 (31.5%) | 12 (29.3%) |
| Female | 71 (75.5%) | 37 (88.1%) | 65 (67.7%) | 37 (68.5%) | 29 (70-7%) |

**Table 6:**

| Screening Colonoscopy Baseline Characteristics | | | | | |
|---|---|---|---|---|---|
| | PBO 4w | RFX 275 2w | RFX 550 2w | RFX 1100 2w | RFX 550 4w |
| | (N=94) | (N=42) | (N=96) | (N=54) | (N=41) |
| | n (%) | n (%) | n (%) | n (%) | n (%) |
| Baseline Abdominal Pain Score | | | | | |
| Not at all | 0 | 0 | 1 (1.0%) | 1 (1.9%) | 0 |
| Hardly | 4 (4.3%) | 3 (7.1%) | 7 (7.3%) | 0 | 2 (4.9%) |
| Somewhat | 18 (19.1%) | 6 (14.3%) | 24 (25.0%) | 9 (16.7%) | 4 (9.8%) |
| Moderately | 32 (34.0%) | 15 (35.7%) | 24 (25.0%) | 17 (31.5%) | 13 (31.7%) |
| A good deal | 26 (27.7%) | 13 (31.0%) | 27 (28.1%) | 17 (31.5%) | 13 (31-7%) |
| A great deal | 14 (14.9%) | 5 (11.9%) | 10 (10.4%) | 10 (18.5%) | 6 (14.6%) |
| A very great deal | 0 | 0 | 3 (3.1%) | 0 | 3 (7.3%) |
| | | | | | |
| Baseline Bloating Discomfort Score | | | | | |
| Not at all | 1 (1.1%) | 0 | 1 (1.0%) | 1 (1.9%) | 1 (2.4%) |
| Hardly | 8 (8.5%) | 3 (7.1%) | 8 (8.3%) | 1 (1.9%) | 1 (2.4%) |
| Somewhat | 17 (16.1%) | 9 (21.4%) | 17 (17.7%) | 11 (20.4%) | 4 (9.8%) |
| Moderately | 22 (23.4%) | 15 (35.7%) | 26 (27.1%) | 14 (25.9%) | 14 (34.1%) |
| A good deal | 29 (30.9%) | 10 (23.8%) | 28 (29.2%) | 17 (31.5%) | 6 (14.6%) |
| A great deal | 15 (16.0%) | 5 (11.9%) | 10 (10.4%) | 9 (16.7%) | 9 (22.0%) |
| A very great deal | 2 (2.1%) | 0 | 6 (6.3%) | 1 (1.9%) | 6 (14.6%) |

**Table 7:**

| Adhoc Table 20 | | | | | | |
|---|---|---|---|---|---|---|
| Efficacy Analysis of Screening Colonoscopy: | | | | | | |
| Adequate Relief of IBS Symptoms and Bloating at the End of the Treatment Phase | | | | | | |
| | | PBO 4w | RFX 275 2w | RFX 550 2w | RFX 1100 2w | RFX 550 4w |
| | | (N=94) | (N=42) | (N=96) | (N=54) | (N-41) |
| | | n (%) | n (%) | n (%) | n (%) | n (%) |
| Average Bloating Discomfort Score at Baseline | | | | | | |
| <=4 | Adequate Relief of IBS Symptoms [1] | | | | | |
| | Success | 32 (41.6%) | 16 (43.2%) | 46 (57.5%) | 23 (52.3%) | 11 (42.3%) |
| | Failure | 45 (58.4%) | 21 (56.8%) | 34 (42.5%) | 21 (47.7%) | 15 (57.7%) |
| | Adequate Relief of Bloating [2] | | | | | |
| | Success | 30 (39.0%) | 15 (40.5%) | 44 (55.0%) | 23 (52.3%) | 11 (42.3%) |
| | Failure | 47 (61.0%) | 22 (59.5%) | 36 (45.0%) | 21 (47.7%) | 15 (57.7%) |
| | | | | | | |
| >4 | Adequate Relief of IBS Symptoms [1] | | | | | |
| | Success | 10 (58.8%) | 3 (60.0%) | 8 (50.0%) | 0 | 8 (53.3%) |
| | Failure | 7 (41.2%) | 2 (40.0%) | 8 (50.0%) | 10 (100.0%) | 7 (46.7%) |
| | Adequate Relief of Bloating [2] | | | | | |
| | Success | 8 (47.1%) | 3 (60.0%) | 8 (50.0%) | 0 | 7 (46.7%) |
| | Failure | 9 (52.9%) | 2 (40.0%) | 8 (50.0%) | 10 (100.0%) | 8 (53.3%) |

| | | | | | | |
|---|---|---|---|---|---|---|
| [1] Subjects achieved success if they reported a 'yes' response to whichever question about IBS symptoms was posed by the IVR system(e.g. adequate relief or control) for =2 out of the 3 final treatment weeks. [2] Subjects achieved success if they reported a 'yes' response to whichever question about symptoms of bloating was posed by the IVR system (e.g. adequate relief or control) for =2 out of the 3 final treatment weeks. | | | | | | |

**Table 8:**

| Efficacy Analysis of Subjects who Had a Screening Colonoscopy: | | | | | | |
|---|---|---|---|---|---|---|
| Adequate Relief of IBS Symptoms and Bloating at the End of the Treatment Phase | | | | | | |
| | | PBO 4w | RFX 275 2w | RFX 550 2w | RFX 1100 2w | RFX 550 4w |
| | | (N=94) | (N=42) | (N=96) | (N=54) | (N=41) |
| | | n (%) | n (%) | n (%) | n (%) | n (%) |
| Average Abdominal Pain Discomfort Score at Baseline | | | | | | |
| <=4 | Adequate Relief of IBS Symptoms [1] | | | | | |
| | Success | 33 (41.3%) | 16 (43.2%) | 46 (55.4%) | 21 (47.7%) | 13 (40.6%) |
| | Failure | 47 (58.8%) | 21 (56.8%) | 37 (44.6%) | 23 (52.3%) | 19 (59.4%) |
| | Adequate Relief of Bloating [2] | | | | | |
| | Success | 30 (37.5%) | 15 (40.5%) | 45 (54.2%) | 22 (50.0%) | 13 (40.6%) |
| | Failure | 50 (62.5%) | 22 (59.5%) | 38 (45.8%) | 22 (50.0%) | 19 (59.4%) |
| >4 | Adequate Relief of IBS Symptoms [1] | | | | | |
| | Success | 9 (64.3%) | 3 (60.0%) | 8 (61.5%) | 2 (20.0%) | 6 (66.7%) |
| | Failure | 5 (35.7%) | 2 (40.0%) | 5 (38.5%) | 8 (80.08) | 3 (33.3%) |
| | Adequate Relief of Bloating [2] | | | | | |
| | Success | 8 (57.1%) | 3 (60.0%) | 7 (53.8%) | 1 (10.0%) | 5 (55.6%) |
| | Failure | 6 (42.9%) | 2 (40.0%) | 6 (46.2%) | 9 (90.0%) | 4 (44.4%) |

| | | | | | | |
|---|---|---|---|---|---|---|
| [1] Subjects achieved success if they reported a 'yes' response to whichever question about IBS symptoms was posed by the IVR system(e.g. adequate relief or control) for =2 out of the 3 final treatment weeks. [2] Subjects achieved success if they reported a 'yes' response to whichever question about symptoms of bloating was posed by the IVR system (e.g. adequate relief or control) for =2 out of the 3 final treatment weeks. | | | | | | |

A study is designed to evaluate the efficacy of a 14-day course of oral rifaximin at 550 mg TID in providing adequate relief from diarrhea-associated IBS (dIBS) symptoms over four weeks. The study populations includes subjects diagnosed with dIBS according to Rome III criteria utilizing the subtype for dIBS from the Rome II criteria. Subjects have a mean abdominal pain and discomfort score < 4.5, a mean bloating score of < 4.5 and a mean stool score (loose and watery) < 3.5 for at least 7 of the 10 days during screening. A measure of efficacy is based on subjects' answers to the Weekly Subject Global Assessment (SGA) questions over the 4 week study duration in relation to their IBS symptoms. The SGA question is asked weekly as follows: *"In the past 7 days, have you had adequate relief of your IBS symptoms? (Yes*/*No.)* It is discovered that Subjects in the treatment group taking oral rifaximin respond "Yes" more often than Subjects who are not taking oral rifaximin. Another measure of efficacy is based on subjects' answers to the Weekly Subject Global Assessment (SGA) question over the 4 week study duration in relation to their IBS symptom of bloating. The SGA question is asked weekly as follows: *"In the past 7 days, have you had adequate relief of your IBS symptom of bloating? " (Yes*/*No).* It is discovered that Subjects in the treatment group taking oral rifaximin respond "Yes" more often than Subjects who are not taking oral rifaximin. Other measures of efficacy include the changes in dIBS symptoms from baseline to each week of the 4 weeks in the study (e.g., abdominal pain and discomfort, bloating, number of stools per day, stool consistency, urgency with loose or watery stools).

In another example, subjects are randomized based on subject responses to the Weekly SGA questions and daily IBS symptoms question asked during a period of 10± 3 days. If a colonoscopy is required, then the diary data will be initiated a minimum of 7 days after the colonoscopy has been performed.

## Claims

1. Use of a gastrointestinal (GI) cleanser and a therapeutically effective amount of rifaximin for the manufacture of a medicament for treating bowel disease (BD) comprising:
administering the gastrointestinal (GI) cleanser to a subject in need thereof; and
administering the therapeutically effective amount of rifaximin, wherein the administration of the gastrointestinal (GI) cleanser is within between about 1 to about 90 days before the administration of the rifaximin.

2. The use of claim 1, wherein the GI cleanser and the rifaximin result in adequate relief of one or more of IBS symptoms, abdominal pain symptoms, or bloating symptoms in about 35 - about 70% of subjects administered the GI cleanser and the rifaximin.

3. The use of claim 1, wherein the GI cleanser comprises one or more of a PEG based composition and a sodium phosphate based composition, or wherein the GI cleanser comprises polyethylene glycol (PEG), sodium sulfate, sodium chloride, potassium chloride and ascorbic acid.

4. The use of claim 1, wherein the use further comprises administering an antibiotic prior to or with the administration of the gastrointestinal cleanser.

5. The use of Claim 4, wherein the antibiotic administered prior to or with administration of the gastrointestinal, cleanser is at least one selected from the group of: rifamycin, aminoglycoside, amphenicol, ansamycin, β-Lactam, carbapenem, cephalosporin, cephamycin, monobactam, oxacephem, lincosamide, macrolide, polypeptide, tetracycline, or a 2,4-diaminopyrimidine class antibiotic.

6. The use of claim 1 or 4, wherein the use further comprises performing a colonoscopy on the subject after the administration of the gastrointestinal cleanser.

7. The use of claim 1, wherein one or more of an anti-inflammatory, one or more additional antibiotics, crofelemer, or metoclopramide is to be administered to the subject.

8. The use of claim 1, wherein the bowel disease comprises, one or more of inflammatory bowel disease (IBD), Crohn's disease, enteritis, colitis, irritable bowel syndrome (IBS), travelers' diarrhea, small intestinal bacterial overgrowth, uncontrolled diarrhea-associated irritable bowel syndrome (dIBS ), or diarrhea-associated irritable bowel syndrome (dIBS).

9. The use of claim 1, wherein the therapeutically effective amount of the rifaximin comprises from between about 100 mg and about 6000 mg; from between about 50 mg and about 2500 mg BID; from between about 50 mg and about 2000 mg TID; 550 mg TID; 550 mg BID; 600 mg TID; 600 mg BID; 1650 mg QD; 200 mg TID; 200 mg BID or 200 mg QD.

10. The use of claim 2, wherein symptoms comprise one or more of overall BD symptoms or bloating.

11. A therapeutic composition comprising a gastrointestinal (GI) cleanser and a therapeutically effective amount of rifaximin for use in a method of treating bowel disease (BD) in a subject in need thereof, wherein the method comprises:
administering the gastrointestinal (GI) cleanser to a subject in need thereof; and administering the therapeutically effective amount of rifaximin, wherein the administration of the gastrointestinal (GI) cleanser is within between about 1 to about 90 days before the administration of the rifaximin.

12. The composition for use according to claim 11, wherein the therapeutically effective amount of the rifaximin comprises from between about 100 mg and about 6000 mg; from between about 50 mg and about 2,500 mg BID; from between about 50 mg and about 2000 mg TID; 550 mg TID; 550 mg BID; 600 mg TID; 600 mg BID; 1650 mg QD; 200 mg TID; 200 mg BID or 200 mg QD.

13. The composition for use according to claim 11, wherein GI cleanser comprises one or more of a PEG based composition or a sodium phosphate based composition.

## Patentansprüche

1. Verwendung eines Magen-Darm- (MD-) Reinigers und einer therapeutisch wirksamen Menge Rifaximin zur Herstellung eines Medikaments zur Behandlung von Darmerkrankung (DE), umfassend:
das Verabreichen des Magen-Darm- (MD-) Reinigers an einen behandlungsbedürftigen Patienten und
das Verabreichen der therapeutisch wirksamen Menge Rifaximin, wobei die Verabreichung des Magen-Darm- (MD-) Reinigers innerhalb von etwa 1 bis etwa 90 Tagen vor der Verabreichung des Rifaximins erfolgt.

2. Verwendung nach Anspruch 1, wobei der MD-Reiniger und das Rifaximin zu einer adäquaten Linderung von einem oder mehreren aus IBS-Symptomen, Bauchschmerzsymptomen oder Blähungssymptomen bei etwa 35 bis etwa 70% der Patienten, denen der MD-Reiniger und das Rifaximin verabreicht worden sind, führt.

3. Verwendung nach Anspruch 1, wobei der MD-Reiniger eine oder mehrere von einer Zusammensetzung auf PEG-Basis und einer Zusammensetzung auf Natriumphosphatbasis umfasst oder wobei der MD-Reiniger Polyethylenglycol (PEG), Natriumsulfat, Natriumchlorid, Kaliumchlorid und Ascorbinsäure umfasst.

4. Verwendung nach Anspruch 1, wobei die Verwendung des Weiteren die Verabreichung eines Antibiotikums vor oder zusammen mit der Verabreichung des Magen-Darm-Reinigers umfasst.

5. Verwendung nach Anspruch 4, wobei das vor oder zusammen mit der Verabreichung des Magen-Darm-Reinigers verabreichte Antibiotikum mindestens eines ist, ausgewählt aus der Gruppe, bestehend aus: Rifamycin, Aminoglycosid, Amphenicol, Ansamycin, β-Lactam, Carbapenem, Cephalosporin, Cephamycin, Monobactam, Oxacephem, Lincosamid, Macrolid, Polypeptid, Tetracyclin oder einem Antibiotikum der 2,4-Diaminopyrimidin-Klasse.

6. Verwendung nach Anspruch 1 oder 4, wobei die Verwendung des Weiteren die Durchführung einer Kolonoskopie bei dem Patienten nach der Verabreichung des Magen-Darm-Reinigers umfasst.

7. Verwendung nach Anspruch 1, wobei ein oder mehrere Entzündungshemmer, ein oder mehrere zusätzliche Antibiotika, Crofelemer oder Metoclopramid an den Patienten zu verabreichen ist.

8. Verwendung nach Anspruch 1, wobei die Darmerkrankung umfasst: eine oder mehrere von entzündlicher Darmerkrankung (IBD), Crohn'scher Erkrankung, Enteritis, Colitis, Reizdarmsyndrom (IBS), Reisediarrhö, bakterielle Überbesiedlung im Dünndarm, durch unkontrollierte Diarrhö bedingtes Reizdarmsyndrom (dIBS) oder Diarrhö-bedingtes Reizdarmsyndrom (dIBS).

9. Verwendung nach Anspruch 1, wobei die therapeutisch wirksame Menge Rifaximin etwa 100 mg bis etwa 6.000 mg; etwa 50 mg bis etwa 2.500 mg BID; etwa 50 mg bis etwa 2.000 mg TID; 550 mg TID; 550 mg BID; 600 mg TID; 600 mg BID; 1.650 mg QD; 200 mg TID; 200 mg BID oder 200 mg QD umfasst.

10. Verwendung nach Anspruch 2, wobei Symptome eines oder mehrere von Gesamt-BD-Symptomen oder Blähung umfassen.

11. Therapeutische Zusammensetzung, umfassend einen Magen-Darm- (MD-) Reiniger und eine therapeutisch wirksame Menge Rifaximin zur Verwendung in einem Verfahren zur Behandlung von Darmerkrankung (DE) bei einem behandlungsbedürftigen Patienten, wobei das Verfahren umfasst:
das Verabreichen des Magen-Darm- (MD-) Reinigers an einen behandlungsbedürftigen Patienten und
das Verabreichen der therapeutisch wirksamen Menge Rifaximin, wobei die Verabreichung des Magen-Darm- (MD-) Reinigers innerhalb von etwa 1 bis etwa 90 Tagen vor der Verabreichung des Rifaximins erfolgt.

12. Zusammensetzung zur Verwendung nach Anspruch 11, wobei die therapeutisch wirksame Menge Rifaximin etwa 100 mg bis etwa 6.000 mg; etwa 50 mg bis etwa 2.500 mg BID; etwa 50 mg bis etwa 2.000 mg TID; 550 mg TID; 550 mg BID; 600 mg TID; 600 mg BID; 1.650 mg QD; 200 mg TID; 200 mg BID oder 200 mg QD umfasst.

13. Zusammensetzung zur Verwendung nach Anspruch 11, wobei der MD-Reiniger eine oder mehrere von einer Zusammensetzung auf PEG-Basis oder einer Zusammensetzung auf Natriumphosphatbasis umfasst.

## Revendications

1. Utilisation d'un agent de nettoyage gastro-intestinal (GI) et d'une quantité thérapeutiquement efficace de rifaximine pour la fabrication d'un médicament destiné au traitement de maladies intestinales (MI) comprenant :
l'administration de l'agent de nettoyage gastro-intestinal (GI) à un sujet en ayant besoin ; et
l'administration de la quantité thérapeutiquement efficace de rifaximine, l'administration de l'agent de nettoyage gastro-intestinal (GI) se situant entre environ 1 et environ 90 jours avant l'administration de la rifaximine.

2. Utilisation selon la revendication 1, où l'agent de nettoyage GI et la rifaximine entraînent un soulagement adéquat d'un ou de plusieurs symptômes du SCI, symptômes de douleurs abdominales, ou symptômes de ballonnement chez environ 35 à environ 70 % des sujets auxquels ont été administrés l'agent de nettoyage GI et la rifaximine.

3. Utilisation selon la revendication 1, où l'agent de nettoyage GI comprend une ou plusieurs d'une composition à base de PEG et d'une composition à base de phosphate de sodium, ou bien où l'agent de nettoyage GI comprend du polyéthylène glycol (PEG), du sulfate de sodium, du chlorure de sodium, du chlorure de potassium et de l'acide ascorbique.

4. Utilisation selon la revendication 1, où l'utilisation comprend en outre l'administration d'un antibiotique avant ou avec l'administration de l'agent de nettoyage gastro-intestinal.

5. Utilisation selon la revendication 4, où l'antibiotique administré avant ou avec l'administration de l'agent de nettoyage gastro-intestinal est au moins l'un choisi dans le groupe de : une rifamycine, un aminoglycoside, un amphénicol, une ansamycine, un β-lactame, un carbapénème, une céphalosporine, une céphamycine, un monobactame, un oxacéphème, un lincosamide, un macrolide, un polypeptide, une tétracycline, ou un antibiotique de la classe des 2,4-diamino-pyrimidines.

6. Utilisation selon la revendication 1 ou 4, où l'utilisation comprend en outre la réalisation d'une colonoscopie chez le sujet après l'administration de l'agent de nettoyage gastro-intestinal.

7. Utilisation selon la revendication 1, où un ou plusieurs d'un anti-inflammatoire, un ou plusieurs antibiotiques supplémentaires, du crofelemer, ou du métoclopramide doivent être administrés au sujet.

8. Utilisation selon la revendication 1, où la maladie intestinale comprend, un ou plusieurs d'une maladie inflammatoire de l'intestin (MII), de la maladie de Crohn, d'une entérite, d'une colite, du syndrome du côlon irritable (SCI), de la diarrhée du voyageur, d'une prolifération bactérienne dans l'intestin grêle, du syndrome du côlon irritable associé à une diarrhée incontrôlée (SCId), ou du syndrome du côlon irritable associé à une diarrhée (SCId).

9. Utilisation selon la revendication 1, où la quantité thérapeutiquement efficace de la rifaximine est comprise entre environ 100 mg et environ 6000 mg ; entre environ 50 mg et environ 2500 mg deux fois par jour ; entre environ 50 mg et environ 2000 mg trois fois par jour ; 550 mg trois fois par jour ; 550 mg deux fois par jour ; 600 mg trois fois par jour ; 600 mg deux fois par jour ; 1650 mg une fois par jour ; 200 mg trois fois par jour ; 200 mg deux fois par jour ou 200 mg une fois par jour.

10. Utilisation selon la revendication 2, où les symptômes comprennent un ou plusieurs des symptômes globaux des MI ou un ballonnement.

11. Composition thérapeutique comprenant un agent de nettoyage gastro-intestinal (GI) et une quantité thérapeutiquement efficace de rifaximine pour une utilisation dans un procédé de traitement de maladies intestinales (MI) chez un sujet en ayant besoin, où le procédé comprend :
l'administration de l'agent de nettoyage gastro-intestinal (GI) à un sujet en ayant besoin ; et
l'administration de la quantité thérapeutiquement efficace de rifaximine, où l'administration de l'agent de nettoyage gastro-intestinal (GI) se situe entre environ 1 et environ 90 jours avant l'administration de la rifaximine.

12. Composition pour une utilisation selon la revendication 11, où la quantité thérapeutiquement efficace de la rifaximine est comprise entre environ 100 mg et environ 6000 mg ; entre environ 50 mg et environ 2500 mg deux fois par jour ; entre environ 50 mg et environ 2000 mg trois fois par jour ; 550 mg trois fois par jour ; 550 mg deux fois par jour ; 600 mg trois fois par jour ; 600 mg deux fois par jour ; 1650 mg une fois par jour ; 200 mg trois fois par jour ; 200 mg deux fois par jour ou 200 mg une fois par jour.

13. Composition pour une utilisation selon la revendication 11, où l'agent de nettoyage GI comprend une ou plusieurs d'une composition à base de PEG ou d'une composition à base de phosphate de sodium.
